# EUROPEAN PATENT SPECIFICATION

(11) **EP 4 168 033 B1**
(45) Date of publication and mention of the grant of the patent: **25.02.2026**
(21) Application number: 21828902.3
(22) Date of filing: 21.06.2021
(51) Int. Cl.: A61K 38/16, A61P 35/00, C07K 14/47, A61K 45/06

(54) **ADMINISTRATION OF CEBP-BETA ANTAGONIST AND METHODS OF USE**
VERABREICHUNG VON CEBP-BETA-ANTAGONISTEN UND VERFAHREN ZUR VERWENDUNG
ADMINISTRATION D'ANTAGONISTE DE CEBP-BÊTA ET MÉTHODES D'UTILISATION

(30) Priority: 21.06.2020 US 202063041989 P; 21.06.2020 US 202063041986 P; 21.06.2020 US 202063041991 P; 21.06.2020 US 202063041988 P; 21.06.2020 US 202063041990 P; 08.04.2021 US 202163172560 P
(43) Date of publication of application: 26.04.2023
(73) Proprietor: Sapience Therapeutics, Inc., Tarrytown, NY 10591 (US)
(72) Inventor: KAPPEL, Barry, Jay, Harrison, NY 10528 (US); MERUTKA, Gene, Harrison, NY 10528 (US); ROTOLO, Jimmy, Andrew, Harrison, NY 10528 (US); MICHEL, Robert E., Harrison, NY 10528 (US); BEXON, Alice Susannah, Harrison, NY 10528 (US)
(74) Representative: Vossius & Partner Patentanwälte Rechtsanwälte mbB
(86) International application number: PCT/US2021/038264
(87) International publication number: WO 2021/262604

(56) References cited:
- WO-A1-2019/136125
- WO-A2-2020/033555
- US-A1- 2016 361 441
- US-A1- 2018 237 499
- US-A1- 2019 201 483
- SAPIENCE THERAPEUTICS: "Sapience Therapeutics Receives United Kingdom Authorization to Initiate a Phase 1/2 Trial of Lead Candidate ST101 in Advanced Cancer Indications", 7 May 2020 (2020-05-07), XP093126430, Retrieved from the Internet <URL:https://sapiencetherapeutics.com/wp-content/uploads/2023/01/2020-05-07_Sapience_Therapeutics_Receives_United_Kingdom_16.pdf> [retrieved on 20240201]
- ROTOLO JIM A ET AL: "ST101 Mechanism of Action ST101 In Vitro Activity Cell penetrating peptide, ST101, disrupts ATF5 regulation of anti-apoptotic Bcl-2 family proteins, resulting in induction of cancer cell death in vitro and tumor growth inhibition/regression in vivo", 1 April 2019 (2019-04-01), XP093126423, Retrieved from the Internet <URL:https://sapiencetherapeutics.com/wp-content/uploads/2022/12/Sapience-Therapeutics-2019-AACR-poster.pdf> [retrieved on 20240201]
- ANONYMOUS: "Record History | ver. 1: 2020-07-15 | NCT04478279 | ClinicalTrials.gov", 15 July 2020 (2020-07-15), XP093126427, Retrieved from the Internet <URL:https://clinicaltrials.gov/study/NCT04478279?tab=history&a=1> [retrieved on 20240201]
- EVANS T R JEFFRY ET AL: "Poster Discussion Session Efficacy proof-of-concept from a phase 1 study of a novel therapeutic peptide, ST101, targeting the oncogenic transcription factor C/EBP[beta] in patients with refractory solid tumors", 1 January 2022 (2022-01-01), XP093126519, Retrieved from the Internet <URL:https://ascopubs.org/doi/pdfdirect/10.1200/JCO.2022.40.16_suppl.3014> [retrieved on 20240201]

## Description

### BACKGROUND

CCAAT-enhancer-binding protein β (C/EBPβ) is a transcription factor with roles in cellular processes such as differentiation, inflammation, cell survival, and metabolism. C/EBPβ is upregulated or overactivated to promote tumor survival and proliferation and to inhibit differentiation in many different cancers, including breast, glioblastoma, prostate, and multiple myeloma (Homma 2006, Kim 2008, Pal 2009, Zahnow 2009, Aguilar-Morante 2011). In addition, C/EBPβ has been shown to inversely correlate with disease prognosis and survival. Due to its roles in the regulation of cell proliferation and differentiation (Lekstrom-Himes 1998; Lamb 2003), and its increased expression or activation in many types of cancer (Oya 2003; Homma 2006; Zahnow 2009), C/EBPβ is considered a potential target for therapeutic intervention. However, due in part to the difficulty in targeting transcription factors, no effective C/EBPβ inhibitors have entered clinical development prior to the present invention.

### SEQUENCE LISTING

This application contains a Sequence Listing, which has been submitted electronically in ASCII format and is hereby incorporated by reference in its entirety. The ASCII copy was created on June 18, 2021, is named Sapience_013_WO1_SL.txt, and is 2,047 bytes in size.

### SUMMARY OF THE INVENTION

The present invention relates to the embodiments as set out in the claims. Particularly, the invention relates to a pharmaceutical composition comprising a peptide antagonist of CCAAT-enhancer-binding protein β (C/EBPβ) for use in treating a solid tumor in a human patient, wherein the peptide antagonist consists of the D-amino acid sequence VAEAREELERLEARLGQARGELKKWKMRRNQFWLKLQR (SEQ ID NO:2), wherein the pharmaceutical composition is to be administered via intravenous infusion, and wherein the peptide antagonist is to be administered at a dose of 0.5-16 mg/kg.

As further detailed below, the D-amino acid sequence set forth in SEQ ID NO:2 is referenced herein as ST101.

Some of the main aspects of the present invention are summarized below. Additional aspects are described in the Detailed Description of the Invention, Examples, Drawings, and Claims sections of this disclosure. The description in each section of this disclosure is intended to be read in conjunction with the other sections.

The disclosure provides a method of treating a solid tumor in a patient, the method comprising parenterally administering to the patient a pharmaceutical composition comprising an effective amount of a peptide antagonist of CCAAT-enhancer-binding protein β (C/EBPβ). Also provided is a pharmaceutical composition for parenteral administration comprising an effective amount of a peptide antagonist of C/EBPβ for use in treating a solid tumor in a patient. The peptide antagonist is preferably administered to the patient at a dose of about 0.5-16 mg/kg. In the context of the invention as disclosed herein, it is understood that such references to "methods of prevention and/or treatment" comprising the administration of a pharmaceutical composition, and analogous terms, are to be understood as directed to the pharmaceutical composition for use in the disclosed prevention and/or treatment.

In one embodiment, the solid tumor is a melanoma, a carcinoma, or a sarcoma. In certain embodiments, the patient has been diagnosed with locally advanced or metastatic breast cancer (LA/MBC), melanoma, glioblastoma (GBM), or castration-resistant prostate cancer (CRPC).

In some instances, the patient has received a previous treatment selected from the group consisting of chemotherapy, hormone-based therapy, immunotherapy, targeted therapy, and combinations thereof. In one embodiment, the previous treatment is a standard treatment (standard-of-care treatment) for the patient's diagnosis.

The claimed peptide antagonist of C/EBPβ comprises the D-amino acid sequence VAEAREELERLEARLGQARGEL (SEQ ID NO: 1). Provided is also a peptide antagonist that comprises the amino acid sequence LEGRAQGLRAELRELEERAEAV (SEQ ID NO: 4). The peptide antagonist can be a cell-penetrating peptide. The peptide antagonist as claimed is ST101.

Particular methods of the disclosure include methods of treating melanoma, HR^{pos} LA/MBC, primary GBM, or CRPC in a patient, the method comprising administering to the patient via intravenous infusion a pharmaceutical composition comprising an effective amount of ST101. Also provided is a pharmaceutical composition for intravenous infusion comprising an effective amount of ST101 for use in treating melanoma, HR^{pos} LA/MBC, primary GBM, or CRPC in a patient.

In preferred embodiments, the patient is a human patient.

In certain embodiments, the patient has received at least one line of therapy prior to administration of ST101.

In one embodiment, the patient has advanced/metastatic melanoma that has progressed after or on treatment with an immune checkpoint inhibitor. In one embodiment the patient has melanoma comprising a BRAF mutation, and wherein the patient has received at least one line of targeted therapy.

In one embodiment, the GBM has recurred or progressed after previous treatment by maximal surgical resection, radiotherapy, and concomitant temozolomide with radiotherapy or adjuvant chemotherapy with temozolomide.

In one embodiment, the patient has CRPC that has progressed after previous treatment with a taxane, abiraterone, daralutamide, and/or enzalutamide/apalutamide.

In certain embodiments, the pharmaceutical composition is administered to the patient intravenously, for example, via infusion. In some embodiments, the total infusion duration is from about 30 minutes to about 360 minutes, or from about 60 minutes to about 360 minutes or from about 60 minutes to about 180 minutes.

In one aspect, the pharmaceutical composition is administered once weekly. In another aspect, the pharmaceutical composition is administered once every two weeks. The pharmaceutical composition can be administered for at least about three weeks or at least about four weeks.

Some embodiments of the invention include administration of one or more secondary agents. For example, the secondary agents are selected from the group consisting of antihistamines, leukotriene inhibitors, nonsteroidal anti-inflammatory drugs, acetaminophen, corticosteroids, antinausea medications, intravenous saline, electrolytes, and combinations thereof. The one or more secondary agent can be administered to the subject concurrently with, before, or after administration of the pharmaceutical composition. In a particular embodiment, an antihistamine and/or a leukotriene inhibitor is administered to the subject within about 48 hours prior to administration of the pharmaceutical composition.

In certain embodiments, the pharmaceutical composition comprises a buffer and a bulking agent. In a particular embodiment, the pharmaceutical composition comprises lactic acid and trehalose. The pH of the pharmaceutical composition can be, for example, about 3.0-8.0.

In some embodiments of the invention, clearance of ST101 is 0.75-3.5 liters per hour. In some embodiments, half-life (t_{1/2}) of ST101 is 10-70 hours.

### BRIEF DESCRIPTION OF THE DRAWINGS

**FIG. 1** shows the mechanism of action of ST101. C/EBPβ drives tumor cell proliferation and survival, and inhibits differentiation in many cell types. ST101 disrupts the interaction of C/EBPβ with cofactors such as ATF5, depriving cells of oncogenic signals and resulting in selective tumor cell death.
**FIG. 2** shows a modeled structure of ST101 (top) bound to C/EBPβ (bottom). The structure was generated using a crystal structure of ATF4 bound to C/EBPβ as a model (PDB ID 1ci6) in Molecular Operating Environment (MOE).
**FIG. 3** shows circular dichroism (CD) spectroscopy indicating that ST101 interacts with C/EBPβ (panel A) but not with ATF5 (panel B). Individual spectra are shown for ST101 (top gray lines, panels A and B), C/EBPβ (light gray line, panel A) and ATF5 (light gray line, panel B). Black lines indicate actual ST101 + C/EBPβ (panel A) or ST101 + ATF5 (panel B) observations, compared to the dashed lines representing the mean of the two respective individual spectra.
**FIG. 4** shows cellular thermal shift assay indicating that ST101 associates with C/EBPβ in U251 human glioblastoma cells. Western blot (panel A) and densitometric quantification (panel B) of C/EBPβ/β-tubulin expression are shown.

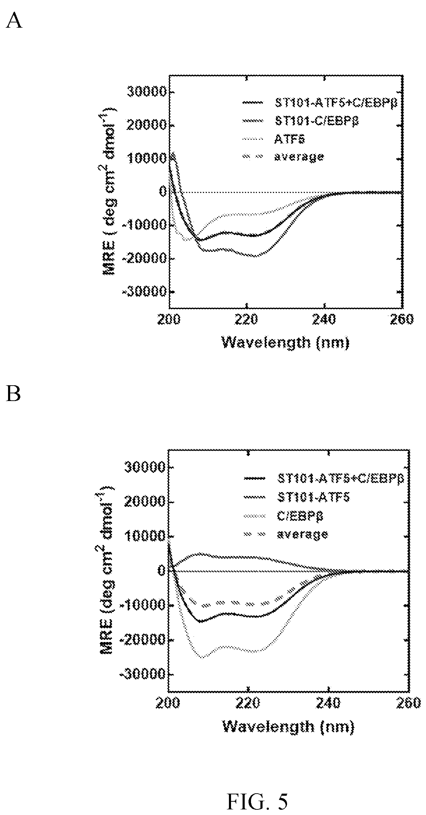

**FIG. 5** shows that C/EBPβ preferentially interacts with ST101 over ATF5, as expressed by molar residue ellipticity (MRE) across a range of wavelengths. Lowest line at 220 nm in panel A shows spectrum of ST101+C/EBPβ; highest line in panel B shows spectrum of ST101+ATF5. Light gray lines show individual spectra of ATF5 (top line at 220 nm in panel A) or C/EBPβ (bottom line at 220 nm in panel B). Black lines show actual spectrum of ST101+C/EBPβ incubated with ATF5 compared to the dashed lines representing the mean of the ST101+C/EBPβ and ATF5 spectra (panel A) or the mean of the ST101+ATF5 and C/EBPβ spectra (panel B).
**FIG. 6** shows that ST101 disrupts C/EBPβ interaction with ATF5. ATF5 binding to C/EBPβ is shown in panel A. ST101 inhibition of ATF5 binding to C/EBPβ is shown in panel B.
**FIG. 7** shows that ST101 crosses the blood-brain barrier in mice. Representative images indicate ST101, identified by DAB (3, 3 -diaminobenzidine) stain, in microvascular and glial cells (arrows, panel B), with no stain present in vehicle-treated controls (panel A).
**FIG. 8** shows the status of the study described in Example 6. PR = partial response (at least 30% decrease); SD = stable disease (less than 30% decrease and 20% increase).
**FIG. 9** shows ST101 plasma concentration after day 1 infusion in patients of Cohorts 1-4 for cycles 1 and 2, as described in Example 6. ST101 was administered at a dose of 0.5 mg/kg (Cohort 1), 1 mg/kg (Cohort 2), 2 mg/kg (Cohort 3), or 4 mg/kg (Cohort 4).
**FIG. 10** shows Cₘₐₓ (panel A) and AUC₀₋ₜ (panel B) of ST101 of individual patients after day 1 infusion of Cohorts 1-4, as described in Example 6. ST101 was administered at a dose of 0.5 mg/kg (Cohort 1), 1 mg/kg (Cohort 2), 2 mg/kg (Cohort 3), or 4 mg/kg (Cohort 4).
**FIG. 11** shows ST101 plasma concentration at four hours post-infusion in patients of Cohorts 1-4, as described in Example 6. ST101 was administered at a dose of 0.5 mg/kg (Cohort 1), 1 mg/kg (Cohort 2), 2 mg/kg (Cohort 3), or 4 mg/kg (Cohort 4).
**FIG. 12** shows the increase in ST101 uptake in tumor biopsies with increasing ST101 dose (panels A-C). Biopsy samples collected from patients during cycle 2 of treatment were processed and immunoassayed for ST101 using rabbit polyclonal anti-ST101 antibody. The percentage of cells exhibiting ST101 staining was determined by Board-certified pathologist (panel D). Scores were assigned based on the number of cells in a sample staining positive for ST101: 0 = negative; 1 = 1-10%; 2 = 11-25%; 3 = 26-50%; 4 = 51-75%; 5 = 76-100%.

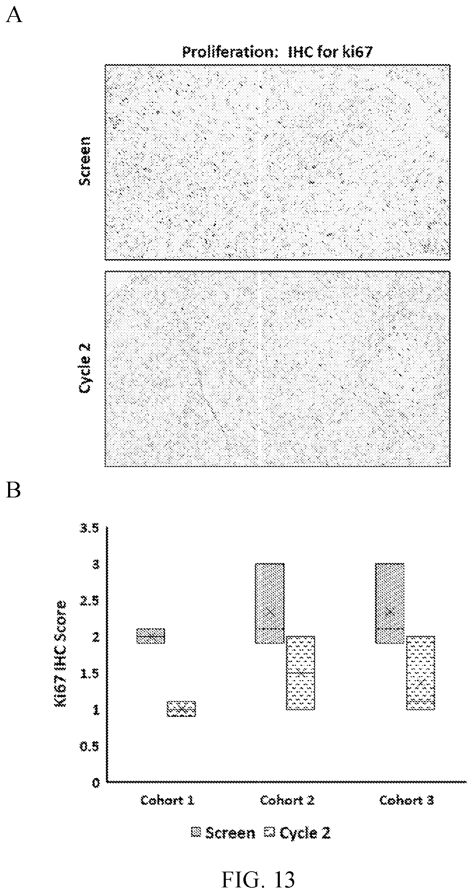

**FIG. 13** shows decreased tumor cell proliferation in patient biopsies following treatment with ST101. Biopsy samples, collected from patients pre-treatment (screen) and after cycle 2 of treatment, were processed and immunoassayed for Ki67. Images in panel A shows staining of biopsy samples from a patient in Cohort 3 prior to treatment (upper image) and after cycle 2 of treatment (lower image). Higher expression of Ki67 indicates greater proliferation of tumor cells. The percentage of cells exhibiting Ki67 staining from patients in each of Cohorts 1-3 was determined by Board-certified pathologist (panel B). Scores were assigned based on the intensity of the Ki67 signal on a scale of 0-3: 0 = negative; 1 = mild; 2 = moderate; 3 = strong.

### DETAILED DESCRIPTION OF THE INVENTION

The practice of the present invention can employ, unless otherwise indicated, conventional techniques of pharmaceutics, formulation science, protein chemistry, cell biology, cell culture, molecular biology, microbiology, recombinant DNA, immunology, clinical pharmacology, and clinical practice, which are within the skill of the art.

In order that the present invention can be more readily understood, certain terms are first defined. Additional definitions are set forth throughout the disclosure. Unless defined otherwise, all technical and scientific terms used herein have the same meaning as commonly understood by one of ordinary skill in the art to which this invention is related.

Any headings provided herein are not limitations of the various aspects or embodiments of the invention, which can be had by reference to the specification as a whole. Accordingly, the terms defined immediately below are more fully defined by reference to the specification in its entirety.

### I. Definitions

The phraseology or terminology in this disclosure is for the purpose of description and not of limitation, such that the terminology or phraseology of the present specification is to be interpreted by the skilled artisan in light of the teachings and guidance.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C; A, B, or C; A or B; A or C; B or C; A and B; A and C; B and C; A (alone); B (alone); and C (alone).

Wherever embodiments are described with the language "comprising," otherwise analogous embodiments described in terms of "consisting of" and/or "consisting essentially of" are included.

Units, prefixes, and symbols are denoted in their Système International d'Unités (SI) accepted form. Numeric ranges are inclusive of the numbers defining the range, and any individual value provided herein can serve as an endpoint for a range that includes other individual values provided herein.

Unless otherwise indicated, the terms "at least" or "about" preceding a series of elements is to be understood to refer to every element in the series. The term "about" preceding a numerical value includes ± 10% of the recited value. For example, a concentration of about 1 mg/mL includes 0.9 mg/mL to 1.1 mg/mL. Likewise, a concentration range of about 1% to 10% (w/v) includes 0.9% (w/v) to 11% (w/v).

The terms "polypeptide," "peptide," and "protein" are used interchangeably to refer to polymers of amino acids of any length, and their salts. The polymer can be linear or branched, can comprise modified amino acids, and can be interrupted by non-amino acids. Except where indicated otherwise, e.g., for the abbreviations for the uncommon or unnatural amino acids set forth herein, the three-letter and one-letter abbreviations, as used in the art, are used herein to represent amino acid residues. Except when preceded with a "D" or in lower case, the amino acid is an L-amino acid. Groups or strings of amino acid abbreviations are used to represent peptides. Except where specifically indicated, peptides are indicated with the N-terminus of the left and the sequence is written from the N-terminus to the C-terminus.

A "retro inverso" peptide has a reversed amino acid sequence, relative to a reference L-amino acid sequence, and is made up of all D-amino acids (inverting the α-center chirality of the amino acid subunits) to help maintain side-chain topology similar to that of the original L-amino acid peptide.

An "isolated" molecule is one that is in a form not found in nature, including those which have been purified.

"Binding affinity" generally refers to the strength of the sum total of non-covalent interactions between a single binding site of a molecule and its binding partner (e.g., a receptor and its ligand, an antibody and its antigen, two monomers that form a dimer, etc.). Unless indicated otherwise, as used herein, "binding affinity" refers to intrinsic binding affinity which reflects a 1:1 interaction between members of a binding pair. The affinity of a molecule X for its partner Y can generally be represented by the dissociation constant (K_{D}). Affinity can be measured by common methods known in the art, including those described herein. Low-affinity binding partners generally bind slowly and tend to dissociate readily, whereas high-affinity binding partners generally bind faster and tend to remain bound longer.

The affinity or avidity of a molecule for its binding partner can be determined experimentally using any suitable method known in the art, *e.g.,* flow cytometry, enzyme-linked immunosorbent assay (ELISA), or radioimmunoassay (RIA), or kinetics (e.g., KINEXA^{®} or BIACORE^{™} or OCTET^{®} analysis). Direct binding assays as well as competitive binding assay formats can be readily employed. (*See, e.g.,* Berzofsky et al., "Antibody-Antigen Interactions," in Fundamental Immunology, Paul, W. E., ed., Raven Press: New York, N.Y. (1984); Kuby, Immunology, W. H. Freeman and Company: New York, N.Y. (1992)). The measured affinity of a particular binding pair interaction can vary if measured under different conditions (e.g., salt concentration, pH, temperature). Thus, measurements of affinity and other binding parameters (e.g., K_{D} or Kd, Kₒₙ, K_{off}) are made with standardized solutions of binding partners and a standardized buffer, as known in the art.

An "active agent" is an ingredient that is intended to furnish biological activity. The active agent can be in association with one or more other ingredients. An active agent that is a peptide can also be referred to as an "active peptide."

An "effective amount" of an active agent is an amount sufficient to carry out a specifically stated purpose.

The term "pharmaceutical composition" refers to a preparation that is in such form as to permit the biological activity of the active ingredient to be effective and which contains no additional components that are unacceptably toxic to a subject to which the composition would be administered. Such composition can be sterile and can comprise a pharmaceutically acceptable carrier, such as physiological saline. Suitable pharmaceutical compositions can comprise one or more of a buffer (*e.g.,* acetate, phosphate, or citrate buffer), a surfactant (*e.g.,* polysorbate), a stabilizing agent (*e.g.,* polyol or amino acid), a preservative (*e.g*., sodium benzoate), and/or other conventional solubilizing or dispersing agents.

A "subject" or "individual" or "animal" or "patient" or "mammal," is any subject, particularly a mammalian subject, for whom diagnosis, prognosis, or therapy is desired. Mammalian subjects include humans, domestic animals, farm animals, sports animals, and laboratory animals including, e.g., humans, non-human primates, canines, felines, porcines, bovines, equines, rodents, including rats and mice, rabbits, etc.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. In certain embodiments, a subject is successfully "treated" for a disease or disorder if the patient shows total, partial, or transient alleviation or elimination of at least one symptom or measurable physical parameter associated with the disease or disorder.

A "control patient" is a subject that has not received a treatment of the invention. A "control population" or a "population of control patients" is a group of subjects that have not received a treatment of the invention. A control patient or subject in the control population has the same disease or disorder as the subject being compared to the control patient or control population. For example, a clinical outcome of a cancer patient receiving a pharmaceutical composition or method of the invention is compared with the average (median) outcome of subjects having the same type of cancer who did not receive a pharmaceutical composition or method of the invention. In some embodiments, the control patient or patients in the control population have received a treatment other than a treatment of the invention, for example, a standard-of-care treatment.

An "antagonist" is a substance that prevents, blocks, inhibits, neutralizes, or reduces a biological activity or effect of another molecule, such as a receptor or ligand.

The terms "inhibit," "block," and "suppress" are used interchangeably and refer to any statistically significant decrease in occurrence or activity, including full blocking of the occurrence or activity. For example, "inhibition" can refer to a decrease of about 10%, 20%, 30%, 40%, 50%, 60%, 70%, 80%, 90% or 100% in activity or occurrence. An "inhibitor" is a molecule, factor, or substance that produces a statistically significant decrease in the occurrence or activity of a process, pathway, or molecule.

A "neoplastic cell" or "neoplasm" typically has undergone some form of mutation/transformation, resulting in abnormal growth as compared to normal cells or tissue of the same type. Neoplasms include morphological irregularities, as well as pathologic proliferation. Neoplastic cells can be benign or malignant. Malignant neoplasms, *i.e.,* cancers, are distinguished from benign in that they demonstrate loss of differentiation and orientation of cells, and have the properties of invasion and metastasis.

A "tumor" or "solid tumor" is a mass of neoplastic cells, such as cancer cells. The terms "advanced," "metastatic," and "advanced/metastatic" are used interchangeably to describe a cancer in which malignant cells have migrated from the original tumor to another location, for example, another organ, in a patient's body.

"Pharmacokinetics" or "PK" refers to the study of how an administered substance is processed by the body of a subject. PK determinations include how the substance enters the blood circulation (absorption), is dispersed or disseminated throughout the fluids and tissues of the body (distribution), is recognized and transformed by the body (metabolism), and is removed from the body (excretion). The substance may be a drug, for example, ST101. Pharmacokinetics may be evaluated using various metrics, many of which are calculated based on the quantity of the substance in the body (*e.g.,* in the plasma) at various time points following the administration of the substance.

Time after administration is measured from T₀, which is the time that administration of a single dose of the substance is started. In instances where administration of the pharmaceutical composition is paused and resumed one or more times during the total infusion period, T₀ is the beginning of the total infusion period.

The "total infusion duration" or "total infusion period" is the time from the start of a single dose of the pharmaceutical composition to the end of administration, and includes both periods of infusion and periods of interruption.

"Cₘₐₓ" is a pharmacokinetic metric that refers to the peak plasma concentration of the substance after administration.

"Tₘₐₓ" is a pharmacokinetic metric that refers to the time after the start of administration of the substance (T₀) to reach Cₘₐₓ.

"Tₗₐₛₜ" is a pharmacokinetic metric that refers to the time of last quantifiable concentration of the substance.

"AUC" or "area-under-the-curve" is a pharmacokinetic metric that describes the variation of the concentration of the substance in blood plasma as a function of time. AUC may be calculated for different periods of time, for example, from time zero to specified time t (AUCₜ or AUC₀₋ₜ), from time zero to infinity (AUC_{∞} or AUC_{0-∞}), etc.

"Elimination half-life" or "half-life" or "t½" is a pharmacokinetic metric that refers to the time required for the concentration of the substance to reach half of its original value.

"Clearance" is a pharmacokinetic metric that refers to the volume of plasma cleared of the substance per unit time.

"V_{z}" is a pharmacokinetic metric that refers to the volume of distribution during terminal phase.

### II. Peptides and Compositions

### C/EBPβ

Transcription factors (TFs) account for approximately 20% of all known oncogenes and are involved in many human cancers (Lambert 2018). Nevertheless, TFs have historically been viewed as "undruggable" targets due to the inability of small molecules to impact their activity or large molecules to reach them intracellularly (Yan 2013; Bushweller 2019). TFs typically dimerize via alpha-helical basic leucine zipper (bZIP) domains, bringing together their DNA-binding domains to allow for efficient interaction with key consensus sequences within the DNA (Asada 2011; Potapov 2015). Disruption of these TF protein-protein interactions (PPIs) represents a potentially powerful approach to drugging this elusive class of targets. Recently, peptides have emerged as a therapeutic class capable of targeting and disrupting TF interactions with high affinity and specificity, with proof-of-concept antagonism demonstrated against multiple previously "undruggable" targets (Takada 2012; Walensky 2014; Bruzzoni-Giovanelli 2018; Lathbridge 2018; Demma 2019; Lathbridge 2019).

C/EBPβ represents a prime target for development of peptide anatagonists, due to its reliance on basic leucine zipper interactions with co-factors. In order to associate with DNA and transactivate gene expressions, C/EBPβ dimerizes with binding partners via interactions between their bZIP domains. In addition to homodimerization, C/EBPβ forms heterodimers with bZIP-containing transcription factors such as Jun/Fos, C/EBPγ (Huggins 2013), delta-interacting protein A (Bezy 2005), and the CREB/ATF family (Zhao 2014).

Activating transcription factor 5 (ATF5) is a CREB/ATF factor that has been determined to associate with and activate C/EBPβ in HEK293 and HCT116 cancer cells, resulting in transactivation of a pro-survival phenotype (Zhang 2015). ATF5 is highly expressed in many cancers, including glioma, where it contributes to the oncogenic phenotype by driving the overexpression of Bcl-2 family proteins and survivin, but is largely not found in differentiated cell types (Sheng 2010). Overexpression of the truncated bZIP domain of ATF5 lacking a DNA-binding domain in glioma and other tumor cells resulted in cancer cell cytotoxicity (Angelastro 2006); administration of a peptide containing the truncated bZIP domain produced similar results (Cates 2016; Karpel-Massler 2016).

### C/EBPβ Antagonist Peptides

Methods of the invention comprise treating a patient having a solid tumor with an effective amount of a peptide antagonist of C/EBPβ that comprises the D-amino acid sequence VAEAREELERLEARLGQARGEL (SEQ ID NO: 1), which is a retro inverso variant of the wild-type ATF5 bZIP domain. Peptide antagonists of C/EBPβ can be designed, for example, as described in Example 1.

The peptide antagonist of C/EBPβ can be a cell-penetrating peptide. In one embodiment, the peptide comprises a cell-penetrating domain. Numerous cell-penetrating peptide sequences are described and characterized in the literature (*see* WO 2019/136125The peptide may be a cyclic peptide. Cyclized peptides, for example, using hydrocarbon staples (Bernal 2007; Bird 2016) or other cyclization methods known in the art, can enter cells via passive diffusion, endocytosis/endosomal escape, or other mechanisms (Dougherty 2019). Peptides can also be delivered to cells via mechanisms that exploit cellular receptors, for example, integrin-targeting, RGD-like sequences. Alternatively, peptides can be encapsulated and delivered to cells in vesicles, such as exosomes or liposomes, or in micelles.

The ability of a peptide based on the native ATF5 bZIP domain to antagonize the activity of C/EBPβ can be measured by methods described herein, for instance, in Example 2. The cytotoxic activity of a peptide antagonist of C/EBPβ can be measured *in vitro* by known assays and/or *in vivo* using known tumor models; for example, WO 2019/136125 describes such assays and models.

ST101 is an all D-amino acid peptide that displays potent anti-tumor activity *in vitro* and *in vivo* and resistance to proteolytic degradation. In particular, we have previously demonstrated cytotoxicity of ST101 in HL60 (promyelocytic leukemia), AML14 (acute myeloid leukemia), SET2 (megakaryoblastic leukemia), A375 (melanoma), MCF7 (breast carcinoma), U87 (glioblastoma), U251 (glioblastoma), DU145 (prostate cancer), A549 (lung cancer), peripheral blood mononuclear cells (PBMC), and bone marrow mononuclear cells (BMMC) (*see* WO 2019/136125). In addition, subcutaneous administration of ST101 in xenograft mouse model using A375, HL60, MCF7, and U251 cells resulted in significant reduction in tumor volume (*see* WO 2019/136125).

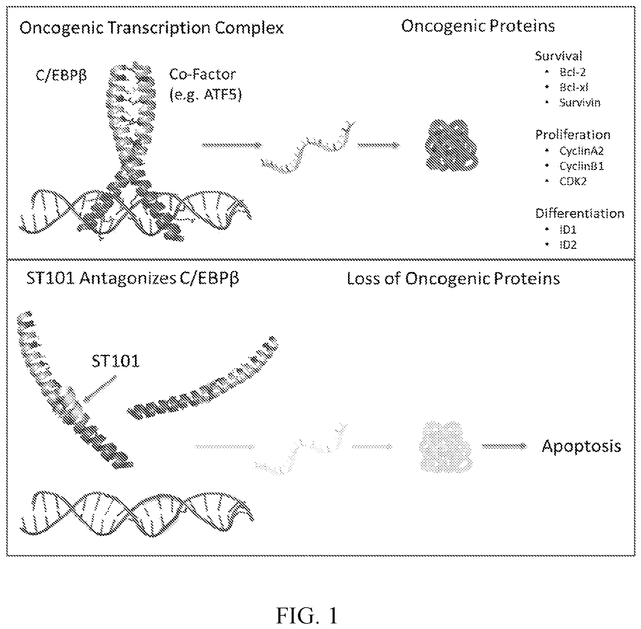

ST101 is comprised of modified domains based on the ATF5 bZIP domain and on the Antennapedia penetratin domain, to allow for cell penetration. The D-amino acid sequence of ST101 is VAEAREELERLEARLGQARGELKKWKMRRNQFWLKLQR (SEQ ID NO: 2), with the cell-penetrating region italicized. As demonstrated herein, ST101 promotes cytotoxic activity in tumor cells by disrupting the association of C/EBPβ with anti-apoptotic transcription factors (**FIG. 1**; Example 2).

### Compositions and Administration

The disclosure provides a pharmaceutical composition, comprising the peptide antagonist of C/EBPβ, ST101. In one embodiment, ST101 can be in a salt form, such as an acetate salt. Preferably, the composition comprises one or more carriers, diluents, excipients, or other additives. For example, the composition can comprise one or more bulking agents (*e.g.,* dextran 40, glycine, lactose, mannitol, trehalose), one or more buffers (*e.g.,* acetate, citrate, histidine, lactate, phosphate, Tris), one or more pH adjusting agents (*e.g*., hydrochloric acid, acetic acid, nitric acid, potassium hydroxide, sodium hydroxide), and/or one or more diluents (*e.g*., water, physiological saline). The pH of the composition is preferably between about 3.0 and 8.0. In one embodiment, the pH is between about 3.5 and 6.5, or between about 5.0 and 7.5.

In some embodiments, the pharmaceutical composition comprises a bulking agent, such as trehalose, in which the amount of the bulking agent is in a particular ratio to the amount of ST101, such as a ratio of bulking agent: ST101 of about 6:1 to about 2:1, by weight.

Aspects of the disclosure relate to methods of administering a peptide antagonist of C/EBPβ to a subject. The peptide antagonist of C/EBPβ is administered parenterally. Parenteral routes of administration include intravenous (IV), intramuscular, intraperitoneal, intrathecal, and subcutaneous. In the claimed embodiments, a pharmaceutical composition comprising ST101 is administered via IV infusion. The pharmaceutical composition can be provided, for example, in an IV fluid comprising normal saline (0.9%), half normal saline (0.45%), 5% dextrose in water (D5W).

The peptide antagonist of C/EBPP is dosed based on the patient's weight. The peptide antagonist of C/EBPβ, such as ST101, can be administered to a patient at a dose of about 0.5 mg/kg to about 16 mg/kg. In certain embodiments, ST101 is administered at a dose of about 0.5 mg/kg, about 0.75 mg/kg, about 1 mg/kg, about 1.5 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 10 mg/kg, about 12 mg/kg, about 14 mg/kg, or about 16 mg/kg. These amounts can also serve as endpoints for a range of doses to be administered, for example, about 0.5 mg/kg to about 8 mg/kg, about 2 mg/kg to about 4 mg/kg, etc.

In embodiments of the invention, the pharmaceutical composition is administered to the subject by intravenous infusion, in which the total infusion duration is no more than about 360 minutes. In some embodiments, the total infusion duration is about 30 minutes to about 240 minutes. For example, the total infusion duration can be 30 minutes, 60 minutes, 90 minutes, 120 minutes, 150 minutes, or 180 minutes, or an intermediate duration, such as 45 minutes, 100 minutes, etc. In certain embodiments, the total infusion duration is about 60 minutes to about 90 minutes, or about 60 minutes to about 120 minutes, or about 90 minutes to about 120 minutes, or about 60 minutes to about 180 minutes.

In some embodiments, infusion of the pharmaceutical composition may be interrupted, *i.e.,* may be temporarily stopped and then resumed. The duration of the interruption may vary, for example, it may be about 15 minutes or less, or about 30 minutes or less, or about one hour or less, or about two hours or less, or about three hours or less, or about four hours or less.

In some embodiments, the pharmaceutical composition may be administered with one or more secondary agents intended to block histamine release, to prevent or ameliorate infusion-related reactions (IRR), to reduce fever or inflammation, and/or to relieve itching and/or urticaria. The one or more secondary agents may be administered concurrently with, before, and/or after the administration of the pharmaceutical composition. The one or more secondary agents may be administered in a separate composition than the pharmaceutical composition, or may be combined with the pharmaceutical composition. In addition, the one or more secondary agents may be administered by the same route as the pharmaceutical composition, or may be administered by a different route (*e*.*g*., orally).

Examples of one or more secondary agents for administration with the pharmaceutical composition include, but are not limited to, antihistamines, including H1 antagonists, H2 antagonists, and mast cell degranulation inhibitors (*e.g.*, acrivastine, astemizole, azatadine, azelastine, bepotastine, bromopheniramine, burfroline, cetirizine, chlorzoxazone, chlorpheniramine, cromolyn, cyproheptadine, desloratadine, dexbromphenir amine, diphenhydramine, doxantrozole, epinastine, etodroxizine, famotidine, fexofenadine, forskolin, hydroxyzine, isoproterenol, ketotifen, levocetirizine, loratadine, lodoxamide, mequitazine, methdilazine, mizolastine, nedocromil, olopatadine, oxatomide, pemirolast, pimecrolimus, pirbuterol, pizotifen, proxicromil, ranitidine, terfenadine, terbutaline); leukotriene inhibitors (*e.g.,* montelukast, zafirlukast, zileuton); nonsteroidal anti-inflammatory drugs (NSAIDs) (*e.g.,* ibuprofen, naproxen, aspirin); acetaminophen/paracetamol; corticosteroids (*e.g.,* hydrocortisone, dexamethasone, prednisone, prednisolone); antinausea medications (*e.g.,* prochlorperazine, ondansetron); and saline and/or electrolytes. In one preferred embodiment, the secondary agent is an antihistamine, such as chlorpheniramine or diphenhydramine. In particular embodiments, the secondary agent is selected from the group consisting of acetaminophen/paracetamol, an H1 antagonist, and H2 antagonist, montelukast, an antiemetic, and combinations thereof.

In certain embodiments, the secondary agent is administered during administration of the pharmaceutical composition, such as during the infusion period. In certain embodiments, the secondary agent, such as an antihistamine, is administered to the subject prior to administration of the pharmaceutical composition, such as within about 7 days prior, or within about 6 days prior, or within about 5 days prior, or within about 4 days prior, or within about 72 hour prior, or about 48 hours prior, or about 24 hours prior, or about 8-12 hours prior, or about 6-8 hours prior, or about 4-6 hours prior, or about 2-4 hours prior, or about 1-2 hours prior, or within about 1 hour prior, or immediately prior. In some embodiments, the secondary agent is administered within 24 hours after administration of the pharmaceutical composition. For example, the secondary agent can be administered immediately after, about 0.5-1 hour after, about 1-2 hours after, about 2-4 hours after, about 4-6 hours after, about 6-8 hours after, about 8-12 hours after, or about 24 hours after the completion of administration of the pharmaceutical composition.

The secondary agent can be administered multiple times before, during, and/or after administration of the pharmaceutical composition. Combinations of secondary agents can be administered concurrently or at different times. For example, an antihistamine could be administered before administration of the pharmaceutical composition, and a corticosteroid could be administered after administration of the pharmaceutical composition.

In one embodiment, administration of the peptide antagonist of C/EBPβ can occur once weekly for a duration of at least three weeks (*i.e.,* three administrations), six weeks (*i.e.,* six administrations), nine weeks, twelve weeks, three months, six months, nine months, or twelve months. In another embodiment, administration can occur once every two weeks for a duration of at least four weeks (*i.e.,* two administrations), eight weeks (*i.e.,* four administrations), twelve weeks, three months, six months, nine months, or twelve months. In some embodiments, a patient can be administered the peptide antagonist of C/EBPβ once weekly for a duration of at least three weeks, six weeks, nine weeks, twelve weeks, three months, six months, nine months, or twelve months, followed by administration once every two weeks for at least four weeks, eight weeks, twelve weeks, three months, six months, nine months, or twelve months.

Pharmacokinetics (PK) of the antagonist of C/EBPβ can be assessed by standard methods and as described in the Examples. In claimed embodiments, the antagonist of C/EBPβ is ST101. The clearance of ST101 can be about 0.75 L/hr to about 3.5 L/hr or about 1 L/hr to about 3 L/hr or about 1 L/hr to about 2.5 L/hr. The half-life (t_{1/2}) of ST101 can be about 10 hours to about 70 hours or about 20 hours to about 60 hours or about 25 hours to about 45 hours.

In some embodiments, the PK are dose proportionate. For example, in patients administered 0.5 mg/kg ST101, the Cₘₐₓ can be about 1,000 ng/mL to about 2,000 ng/mL or about 1,300 ng/mL to about 1,800 ng/mL or about 1,370 ng/mL to about 1,770 ng/mL. In patients administered 1.0 mg/kg ST101, the Cₘₐₓ can be about 2,500 ng/mL to about 5,500 ng/mL or about 2,670 ng/mL to about 5,300 ng/mL or about 2,840 ng/mL to about 5,110 ng/mL. In patients administered 2.0 mg/kg ST101, the Cₘₐₓ can be about 8,000 ng/mL to about 13,000 ng/mL or about 8,500 ng/mL to about 12,500 ng/mL or about 9,000 ng/mL to about 11,900 ng/mL. In patients administered 4.0 mg/kg ST101, the Cₘₐₓ can be about 7,000 ng/mL to about 35,000 ng/mL or about 7,650 ng/mL to about 32,900 ng/mL or about 9,150 ng/mL to about 30,000 ng/mL, or about 15,000 ng/mL to about 28,000 ng/mL.

### III. Methods of Treatment

Subjects in need of treatment by the methods of the invention are patients diagnosed with a solid tumor. For example, the subject can have a locally advanced solid tumor or a metastatic inoperable tumor. In some embodiments, the subject has a melanoma, carcinoma, or sarcoma. In one embodiment, the melanoma is a cutaneous melanoma or a mucosal melanoma. In one embodiment, the carcinoma is adenocarcinoma, such as bladder adenocarcinoma, colorectal adenocarcinoma, pancreatic adenocarcinoma, gastric/signet ring adenocarcinoma, or small bowel adenocarcinoma. In one embodiment, the sarcoma is abdominal sarcoma or myofibroblastic sarcoma. In particular embodiments of the invention, administration of ST101 can inhibit tumor growth, reduce tumor volume, or a combination thereof.

In one embodiment, the patient has locally advanced or metastatic breast cancer (LA/MBC). In one embodiment, the LA/MBC is hormone receptor-positive (HR^{pos}). In one embodiment, the patient has LA/MBC that has progressed after receiving one or two prior hormone-based therapies. In one embodiment, the patient has received previous treatment with a targeted therapy, such as a cyclin-dependent kinase 4/6 (CDK4/6) inhibitor or a mammalian target of rapamycin (mTOR) inhibitor, or with chemotherapy, or with a combination thereof.

In one embodiment, the patient has been diagnosed with melanoma. In one embodiment, the patient has melanoma that has progressed after receiving one or two prior lines of therapy for melanoma, particularly advanced/metastatic melanoma.

In one embodiment, the patient has melanoma that has progressed after/or on treatment with an immune checkpoint inhibitor (CPI) or a combination of CPIs. Immune CPIs include, for example, CTLA-4 inhibitors, such as ipilimumab and tremelimumab; PD-1 inhibitors, such as cemiplimab, nivolumab, pembrolizumab, pidilizumab, and spartalizumab; PD-L1 inhibitors, such as atezolizumab, avelumab, and durvalumab; LAG-3 inhibitors, such as relatilimab; TIGIT inhibitors, such as tiragolumab; and agents that target toll-like receptor 9 (TLR9), such as TLR9 agonists.

In one embodiment, the patient has BRAF-mutated disease and has received at least one line of targeted therapy, such as a BRAF inhibitor and/or a MEK inhibitor, for example, vemurafenib, dabrafenib, encorafenib, trametinib, cobimetinib, binimetinib, or combinations thereof.

In one embodiment, the patient has been diagnosed with glioblastoma (GBM). In one embodiment, the GBM has recurred or progressed after receiving a previous line of therapy. Previous lines of therapy include, for example, surgery, radiation, chemotherapy, targeted therapy, electric field therapy, and combinations thereof. Chemotherapeutic compounds include, for example, temozolomide, carmustine, and lomustine. Targeted therapy includes, for example, bevacizumab.

In one embodiment, the GBM is primary (de novo) GBM that has recurred or progressed (per modified RANO criteria) after one standard treatment regimen. A "standard treatment regimen" is defined as maximal surgical resection, radiotherapy, and concomitant temozolomide with radiotherapy or adjuvant chemotherapy with temozolomide. In one embodiment, patients have undergone tumor treating fields as an adjuvant to first line therapy.

In one embodiment, the patient has been diagnosed with prostate cancer. In one embodiment, the prostate cancer is castration-resistant prostate cancer (CRPC). "Castration-resistant prostate cancer" (CRPC) is defined by disease progression, for example, per Prostate Cancer Clinical Trials Working Group 3 (PCWG3) criteria (Scher 2016), despite androgen depletion therapy. In some embodiments, the patient has CRPC that has progressed after treatment selected from the group consisting of chemotherapy, such as taxanes, including docetaxel and cabazitaxel, optionally in combination with a a corticosteroid, such as prednisone or prednisolone; immunotherapy, including sipuleucel-T; hormone-based therapy, including abiraterone, enzalutamide, daralutamide, and apalutamide; and combinations thereof. In one embodiment, the patient has CRPC that has progressed after previous treatment with taxanes, abiraterone, daralutamide, and/or enzalutamide/apalutamide.

Efficacy of treatment can be evaluated by one or more known measures. For example, patients subjected to methods of the invention can experience outcomes including extended survival, improved progression-free survival, improved duration of response, longer remission, reduced risk of relapse, and/or improved tumor response to treatment with a peptide antagonist of C/EBPβ, compared with the same outcome(s) in patients not subjected to methods of the invention, *i.e.,* control patients. An outcome in a patient treated by a method of the invention can be compared, for example, to the median outcome in a population of control patients. The population of control patients can be administered, for example, a regimen selected from the group consisting of a placebo, surgery, radiation, chemotherapy, immunotherapy, hormone-based therapy, targeted therapy, and combinations thereof. Comparisons can be analyzed statistically using, for example, the Wilcoxon signed rank test or the Kaplan-Meier method.

Outcome in a patient receiving a peptide antagonist of C/EBPβ, such as ST101, optionally in combination with a standard-of-care treatment, may be compared with median outcome in control patients receiving a placebo. , Outcome in a patient receiving a peptide antagonist of C/EBPβ, such as ST101, may be compared with median outcome in control patients receiving a standard-of-care treatment.

In one embodiment, outcome in a LA/MBC patient receiving ST101, optionally in combination with chemotherapy, is compared with median outcome in LA/MBC patients receiving chemotherapy. In one embodiment, outcome in a LA/MBC patient receiving ST101, optionally in combination with a hormone-based therapy is compared with median outcome in LA/MBC patients receiving hormone-based therapy. In one embodiment, outcome in a LA/MBC patient receiving ST101 is compared with median outcome in LA/MBC patients receiving targeted therapy.

In one embodiment, outcome in a melanoma patient receiving ST101, optionally in combination with chemotherapy, is compared with median outcome in melanoma patients receiving chemotherapy. In one embodiment, outcome in a melanoma patient receiving ST101, optionally in combination with immunotherapy, is compared with median outcome in melanoma patients receiving immunotherapy. In one embodiment, outcome in a melanoma patient receiving ST101, optionally in combination with targeted therapy, is compared with median outcome in melanoma patients receiving targeted therapy.

In one embodiment, outcome in a GBM patient receiving ST101. optionally in combination with one or more of surgery, radiation, chemotherapy, and targeted therapy, is compared with median outcome in GBM patients receiving surgery, radiation, chemotherapy, targeted therapy or a combination thereof. In one embodiment, outcome in a GBM patient receiving ST101, optionally in combination with a standard treatment regimen, is compared with median outcome in GBM patients receiving a standard treatment regimen.

In one embodiment, outcome in a prostate cancer patient receiving ST101, optionally in combination with chemotherapy, is compared with median outcome in prostate cancer patients receiving chemotherapy. In one embodiment, outcome in a prostate cancer patient receiving ST101, optionally in combination with immunotherapy, is compared with median outcome in prostate cancer patients receiving immunotherapy. In one embodiment, outcome in a prostate cancer patient receiving ST101, optionally in combination with hormone-based therapy, is compared with median outcome in prostate cancer patients receiving hormone-based therapy. In one embodiment, outcome in a prostate cancer patient receiving ST101, optionally in combination with taxanes, abiraterone, daralutamide, and/or enzalutamide/apalutamide, is compared with median outcome in prostate cancer patients receiving taxanes, abiraterone, daralutamide, and/or enzalutamide/apalutamide.

Response to treatment compares one or more measures of efficacy after a treatment regimen, as compared to baseline, e.g., prior to treatment with ST101. A baseline assessment is preferably performed within 24, 48, or 72 hours, or within 1, 2, 3, or 4 weeks prior to the first treatment with a peptide antagonist of C/EBPβ. In a one preferred embodiment, a baseline assessment is performed within 24 hours prior to the first ST101 treatment.

In embodiments in which the subject is a prostate cancer patient, baseline and response to treatment can be measured by factors including, for example, evaluation of blood-based biomarkers, such as testosterone, prostate-specific antigen (PSA), and serum cytokines imaging, such as by computed tomography (CT), including contrast-enhanced CT, or magnetic resonance imaging (MRI), including cross-sectional MRI; biopsy/histological analysis; and patient-reported factors, such as pain, analgesic use, physical functioning, quality of life, etc. These parameters can be determined, for example, in accordance with PCWG3 guidelines (Scher 2016).

The PCWG3 paradigm defines two general categories of treatment response: (1) the extent to which the treatment controls, relieves, or eliminates manifestations of disease that were present at baseline; and (2) the extent to which the treatment can prevent or delay future manifestations of disease (Scher 2016; Scher 2011). Accordingly, in one aspect, the invention provides a method of controlling, relieving, or eliminating at least one manifestation of prostate cancer in a patient, including a CRPC patient, the method comprising administering to the patient the peptide antagonist of C/EBPβ, ST101. In another aspect, the invention provides a method of preventing or delaying at least one manifestation of prostate cancer in a patient, including a CRPC patient, the method comprising administering to the patient the peptide antagonist of C/EBPβ, ST101. In one embodiment, the manifestation of prostate cancer is prevented or delayed for at least 4, 6, 8, 10, or 12 weeks, at least 4, 6, 8, 10, 12, 16, 18, or 24 months, or at least 3, 4, or 5 years. Duration of prevention or delay is measured relative to the median duration of prevention or delay in a control population. Manifestations of prostate cancer include, for example, tumor burden, new metastatic lesions, elevated PSA level, bone pathologies, including incidence of fracture, pain, use of pain relievers, including opiates, and reduced quality of life (Scher 2016; Scher 2011).

"Tumor burden" is the total mass or total size of cancerous tissue in a patient's body. Tumor response can be evaluated by measures including objective response rate, disease control rate, and duration of response. These parameters can be determined, for example, by revised Response Evaluation Criteria in Solid Tumors (RECIST 1.1) (Eisenhauer 2009), by modified response assessment in neuro-oncology (mRANO) (Ellingson 2017), or by PCWG3 guidelines (Scher 2016), depending on the type of tumor.

Objective response rate assesses reduction of tumor size, for example, tumor diameter, which can be determined by clinical examination and/or imaging. Where a patient has multiple tumors, tumor size can optionally be expressed as the average diameter of all tumors or by the sum of diameters of all tumors. Superficial tumors can be measured clinically, for instance, using calipers or by photography and ruler measurement. Imaging methods include computed tomography (CT), typically with contrast; X-ray; magnetic resonance imaging (MRI); and positron emission tomography (PET), such as (18)F-fluorodeoxyglucose PET. In one preferred embodiment, CT is utilized to assess tumor response, for example, in a LA/MBC patient or a melanoma patient. In another preferred embodiment, MRI, such as gadolinium-enhanced MRI, is utilized to assess tumor response, for example, in a GBM patient. Accordingly, in one aspect, the invention provides a method of reducing tumor burden, *i.e.,* tumor mass and/or tumor size, in a patient, the method comprising administering to the patient the peptide antagonist of C/EBPβ, ST101. Reduction in tumor burden is measured relative to baseline.

In certain embodiments, particularly those in which assessment is by RECIST 1.1, disease control rate defines the level of tumor response as complete response (CR), which is the disappearance of tumor(s); partial response (PR), which is a decrease of at least 30%, in the size of tumor(s); stable disease, in which there is no change in the size of tumor(s); or disease progression, which is an increase of at least 20%, in tumor size and/or new lesions.

In some embodiments, disease control rate defines the level of tumor response as complete response (CR), partial response (PR), progressive disease (PD), or stable disease (SD). Table I sets forth the modified RANO guidelines for these disease states, based on changes in tumor measurements relative to baseline, and sustained for at least four weeks. Additional parameters and details are provided in Ellingson 2017.

**Table I**

| **State of Disease** | **Change in Sum of Products of Perpendicular Diameters (Bidimensional Product)** | **Change in Tumor Volume** |
|---|---|---|
| Complete response (CR) | 100% decrease | 100% decrease |
| Partial response (PR) | ≥50% decrease | ≥65% decrease |
| Progressive disease (PD) | ≥25% increase | ≥40% increase |
| Stable disease (SD) | <50% decrease to <25% increase | <65% decrease to <40% increase |

Duration of response is the length of time from the achievement of a response until disease progression, *i.e.,* the period in which a tumor does not grow or spread, or death. Duration of response in patients receiving ST101 treatment can be, for example, at least 4, 6, 8, 10, or 12 weeks, at least 4, 6, 8, 10, 12, 16, 18, or 24 months, or at least 3, 4, or 5 years. Accordingly, in one aspect, the invention provides a method of increasing the duration of response in a patient, the method comprising administering to the patient the peptide antagonist of C/EBPβ, ST101. Increase in duration of response is measured relative to the median duration of response in a control population.

Survival can be assessed as overall survival, *i.e.,* the length of time a patient lives, or as progression-free survival, *i.e.,* the length of time a patient is treated without progression or worsening of the disease. Survival can be measured from the date of diagnosis or from the date that treatment commences. Overall survival, median overall survival, progression-free survival, and median progression-free survival can be calculated, for example, by Kaplan-Meier analysis, based on the response to treatment. Accordingly, in one aspect, the invention provides a method of increasing overall survival in a patient, the method comprising administering to the patient the peptide antagonist of C/EBPβ, ST101. Increase in overall survival is measured relative to the median overall survival in a control population. In another aspect, the invention provides a method of increasing progression-free survival in a patient, the method comprising administering to the patient the peptide antagonist of C/EBPβ, ST101. Increase in progression-free survival is measured relative to the median progression-free survival in a control population.

A patient is successfully treated according to the methods of the invention if the patient experiences or displays at least one of the following outcomes after administration of the peptide antagonist of C/EBPβ, ST101:
- undetectability of the tumor (or at least one tumor, if multiple tumors are present at baseline);
- at least about 10%, 20%, 30%, 40%, 50%, 60%, 70%, 80%, or 90% reduction in tumor size compared to baseline;
- no significant increase (*e.g.,* less than 20%) in tumor size compared to baseline;
- significantly increased duration of response, optionally compared with median duration of response of a population of control patients;
- significantly increased progression-free survival, optionally compared with median progression-free survival of a population of control patients;
- significantly increased overall survival, optionally compared with median overall survival of a population of control patients.

### IV. Methods of Preparation

Peptide antagonists of C/EBPβ can be chemically synthesized, for example, using solid-phase peptide synthesis or solution-phase peptide synthesis, or a combination of both. Synthesis may optionally occur as fragments of the peptide that are subsequently combined either chemically or enzymatically.

Alternatively, peptide antagonists of C/EBPβ can be expressed using recombinant methods. For example, nucleic acid molecules encoding ST101 can be constructed by chemical synthesis using an oligonucleotide synthesizer. Nucleic acid molecules can be designed based on the amino acid sequence of ST101 and selection of those codons that are favored in the host cell in which the recombinant ST101 will be produced. Standard methods can be applied to synthesize a nucleic acid molecule encoding a peptide antagonist of C/EBPβ, such as ST101.

Once prepared, the nucleic acid encoding the peptide can be inserted into an expression vector and operably linked to an expression control sequence appropriate for expression of the peptide in a desired host. In order to obtain high expression levels of the peptide, the nucleic acid can be operably linked to or associated with transcriptional and translational expression control sequences that are functional in the chosen expression host.

A wide variety of expression host/vector combinations can be employed to anyone known in the art. Useful expression vectors for eukaryotic hosts include, for example, vectors comprising expression control sequences from SV40, bovine papilloma virus, adenovirus, and cytomegalovirus. Useful expression vectors for bacterial hosts include known bacterial plasmids, such as plasmids from *E. coli,* including pCR1, pBR322, pMB9 and their derivatives, wider host range plasmids, such as M13, and filamentous single-stranded DNA phages.

Suitable host cells include prokaryotes, yeast, insect, or higher eukaryotic cells under the control of appropriate promoters. Prokaryotes include gram negative or gram positive organisms, for example *E. coli* or bacilli. Higher eukaryotic cells can be established or cell lines of mammalian origin, examples of which include Pichia pastoris, 293 cells, COS-7 cells, L cells, C127 cells, 3T3 cells, Chinese hamster ovary (CHO) cells, HeLa cells, and BHK cells. Cell-free translation systems can also be employed.

Peptides can be purified using methods that include, for example, reverse-phase high-performance liquid chromatography (RP-HPLC), multicolumn countercurrent solvent gradient purification (MCSGP), and ion-exchange chromatography.

### EXAMPLES

### Example 1. Design of Peptide Antagonists of C/EBPβ

Initial designs of a precursor peptide containing a 22-amino acid section of the native ATF5 bZIP domain, LEGECQGLEARNRELKERAESV (SEQ ID NO: 3), and a 16-amino acid penetratin domain resulted in dose-dependent cytotoxicity of HL-60 promyelocytic leukemia cells, with an EC₅₀ value of 17.4±0.5 µM. We hypothesized that modifications designed to enhance electrostatic interactions between the peptide and C/EBPβ would increase potency. A panel of 46 rationally-designed peptides probing the molecular packing of non-leucine amino acids of the peptide antagonist with that of the C/EBPβ bZIP domain were synthesized and screened for cytotoxic activity. The "winner" peptide, displaying greatest cytotoxicity, resulted in an EC₅₀ value of 5.5±0.5 µM, or a 3.2-fold increase in potency vs. the parent peptide, demonstrating that the introduced rational changes improved in vitro peptide anti-tumor activity. The "winner" peptide comprises a bZIP domain sequence having the amino acid sequence LEGRAQGLRAELRELEERAEAV (SEQ ID NO: 4) and a penetratin domain.

As L-amino acid peptides typically lack stability in biological matrices, limiting their pharmacokinetics and therefore their therapeutic potential, we generated an all-D amino acid peptide version of the optimized ATF5 bZIP domain, VAEAREELERLEARLGQARGEL (SEQ ID NO: 1), additionally comprising a penetratin domain. This peptide, ST101, displayed proteolytic resistance when incubated in the presence of pepsin and trypsin at 37° C for 17 hours, whereas the L-enantiomer peptide was 100% degraded within 5 minutes. The minimal loss of ST101 observed in an untreated control was attributed to common peptide chemical degradation that occurs under these conditions.

Cytotoxicity testing of ST101 revealed an EC₅₀ of 4.9±0.2 µM in HL-60 promyelocytic leukemia cells, comparable activity to the L-enantiomer peptide of the same amino acid sequence, demonstrating that while the D-amino acid variant displayed significantly enhanced stability, it did not lose anti-tumor activity.

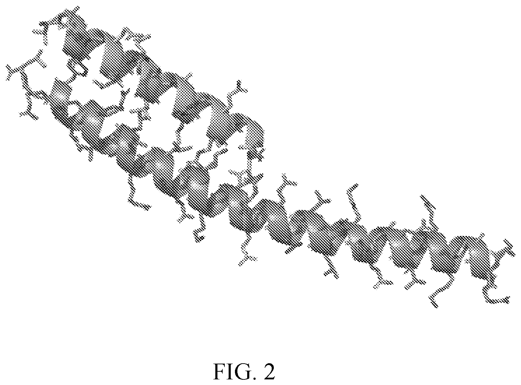

The interaction between ST101 and the C/EBPβ bZIP domain was modeled based on the crystal structure of C/EBPβ and the CREB/ATF family cofactor ATF4 ⁴⁰. ST101 was assumed to be helical in solution. A model of ST101 bound to C/EBPβ was generated using Molecular Operating Environment (MOE) software by constraining the ST101 binding sequence to an alpha helical structure, overlaid with the portion of ATF4 with which it shares the highest sequence similarity, and minimized to favor side-chain interactions. This model indicates that ST101 likely interacts primarily through hydrophobic contacts with some charge-charge (ST101 E12 to C/EBPβ R279 and ST101 E21 to C/EBPβ K267) and cation-pi interactions (ST101 R5 to C/EBPβ F282) (**FIG. 2**).

### Example 2. ST101 Interacts with C/EBPβ but not ATF5

ST101 was shown to interact with C/EBPβ in silico using circular dichroism (CD) spectroscopy. In CD spectroscopy, when peptides/proteins interact, the observed spectrum of the combined sample is different than the mean of the individual spectrum of each component. CD was carried out using an Applied Photophysics Chirascan CD apparatus (Leatherhead, UK) using a 200 µl sample in a CD cell with a 1 mm path length. Samples contained 150 µM total peptide concentration at equimolar concentration for heterodimeric solutions (*i.e.,* 75µM per peptide) and suspended in 10 mM potassium phosphate and 100mM potassium fluoride at pH 7.0 prior to analysis. The CD spectra of samples were scanned between 260 nm and 200 nm in 1 nm steps, averaging 0.5 s at each wavelength. Three scans were averaged at 20°C.

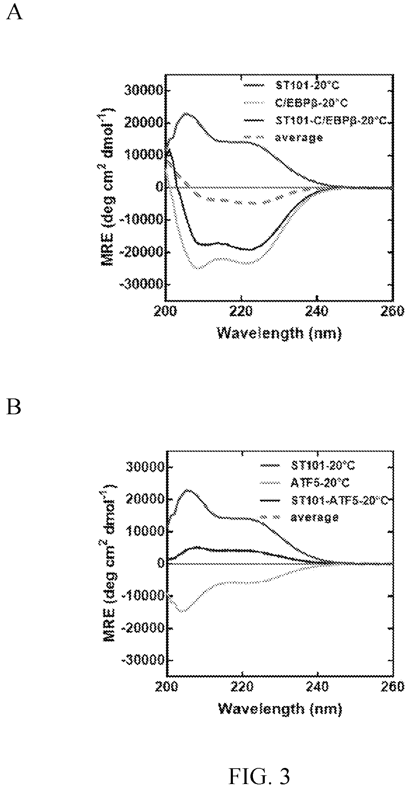

ST101 in combination with a peptide derived from the bZIP domain of C/EBPβ generated a spectrum that differed significantly from the mean of the individual spectra, indicating a structural interaction between the two components (**FIG. 3**, panel A). In contrast, the observed spectrum of ST101 combined with a peptide derived from the bZIP domain of ATF5 did not change from the mean of the individual spectra of the two peptides (**FIG. 3**, panel B), indicating that these peptides do not interact under these conditions. These data demonstrate that ST101 specifically interacts with C/EBPβ.

The interaction of ST101 with C/EBPβ was further demonstrated in a biological setting in U251 human glioblastoma cells using a cellular thermal shift assay (CETSA). Cells were exposed to vehicle or 10 µM ST101 for 1 hour. Lysates were prepared from the cells, and subsequently exposed to a heat gradient spanning 52-60°C to denature proteins. Lysates were then spun down to remove denatured protein aggregates, and seperated by SDS-PAGE. Western blot analysis was performed to detect the C/EBPβ and β-tubulin remaining in the cellular lysates following heating.

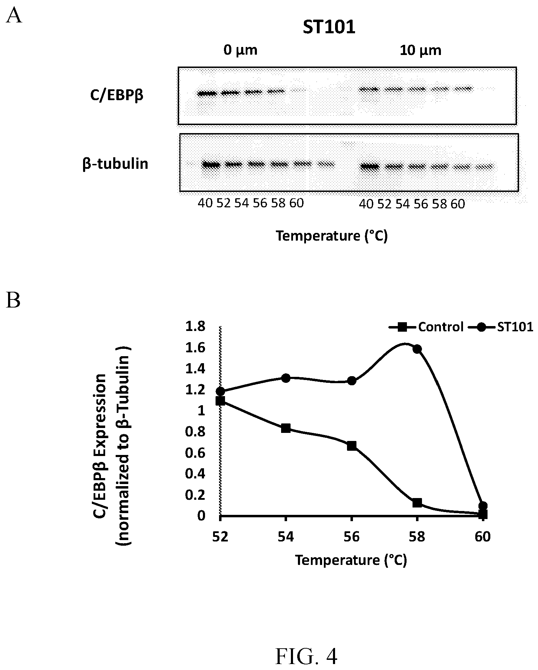

In western blot analysis of heat-denatured cell lysates from untreated U251 cells, denatured C/EBPβ fell out of solution at 58°C, as indicated by loss of the C/EBPβ protein band on the western blot (**FIG. 4**, panel A). Cells treated with 10 µM ST101 displayed increased stability of C/EBPβ at 58°C, as shown by increased detection of C/EBPβ, indicating an interaction between ST101 and C/EBPβ (**FIG. 4**, panels A and B). Control experiments indicated that ST101 did not confer increased stability to ATF5 in the CETSA assay, demonstrating specificity of the interaction with C/EBPβ and consistent with the CD data.

Additional experiments using CD spectroscopy to evaluate dimer exchange demonstrated that C/EBPβ displays greater affinity for ST101 than for ATF5. In these experiments, when ST101 was pre-mixed with a peptide containing the bZIP domain of C/EBPβ, addition of a peptide containing the bZIP domain of ATF5 was unable to displace ST101, as evidenced by no gain of signal (**FIG. 5**, panel A). In contrast, when ST101 was pre-mixed with the ATF5 bZIP peptide, addition of C/EBPβ bZIP peptide resulted in a gain of signal, generating a peak identical to that of ST101 interacting with C/EBPβ bZIP peptide (**FIG. 5****,** panel B). These data demonstrate the preferential interaction of C/EBPβ with ST101 over ATF5, as indicated by a shift in the spectra of C/EBPβ+ATF5 when ST101 is added.

To quantify ST101 inhibition of the C/EBPβ interaction with ATF5, a competitive enzyme-linked immunosorbent assay (ELISA) format was used. C/EBPβ (3.6 ng/well) was immobilized to a 384-well plate (Nunc MaxiSorp, ThermoSci) by overnight incubation at 4°C. Non-bound protein was removed by three washes with tris-buffered saline (TBS) + 0.1% Tween (TBST), and wells were then blocked for 1 hr at 4°C with 5% bovine serum albumin in TBS. After removal of the blocking buffer, ST101 was diluted in TBS and added to the appropriate wells for 1 hr at 4°C, and wells were washed three times with TBST.

Next, 1 ng recombinant ATF5 was added to each well prior to 18-hr incubation at 4°C, and non-bound protein was removed following three washes with TBST. Plate-bound ATF5 was detected by 1-hr incubations at 4°C of 1:1000 dilution rabbit-anti-ATF5 antibody (ab60126, Abcam) followed by 1:1000 goat-anti-rabbit IgG-HRP antibody (ab6721, Abcam), with three washes with TBST after each antibody incubation, and 50 µL TMB substrate. The reaction was stopped by addition of 25 µL 2.5M sulfuric acid, and absorbance was detected at 450 nm using a SpectraMax M3 plate reader (Molecular Devices). ST101 IC₅₀ was calculated by nonlinear regression with four parameters and variable slope using GraphPad Prism 8.3.0 software.

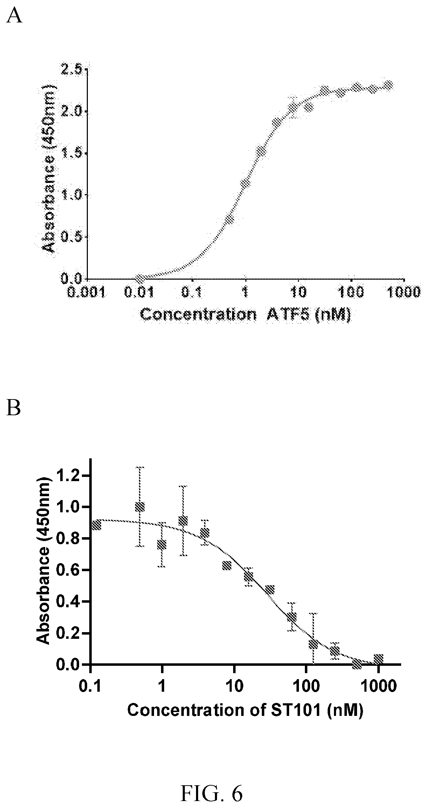

Initial experiments determined that under conditions where 3.6 ng C/EBPβ was plate-bound, ATF5 association resulted in a K_{d} of 1.0 nM (**FIG. 6**, panel A). In subsequent experiments, increasing concentrations of ST101 were added to a constant concentration of 1 nM ATF5, resulting in a dose-dependent decrease in detection of ATF5 (**FIG. 6**, panel B). Nonlinear four parameter analysis determined that the IC₅₀ for ST101 inhibition of ATF5 association with C/EBPβ is 24.6±0.9 nM. These data support the CD dimer exchange experiments.

### Example 3. ST101 Penetrates the Blood-Brain Barrier in Naïve C57BL/6 Mice

To determine whether systemic administration of ST101 can enter the brain in an animal with an intact blood-brain barrier, immunohistochemistry (IHC) analysis of ST101 in naive C57BL/6 brain sections following single systemic administration was performed. ST101 (25 mg/kg) or vehicle was administered by intravenous injection into the lateral tail-vein (n=3/group). Two hours post injection, mice were euthanized. Brains were harvested and stored in 4% paraformaldehyde. Tissues were processed for IHC staining on 2 µM sections, using a rabbit polyclonal anti-ST101 antibody detected with DAB conjugated anti-rabbit secondary reagent.

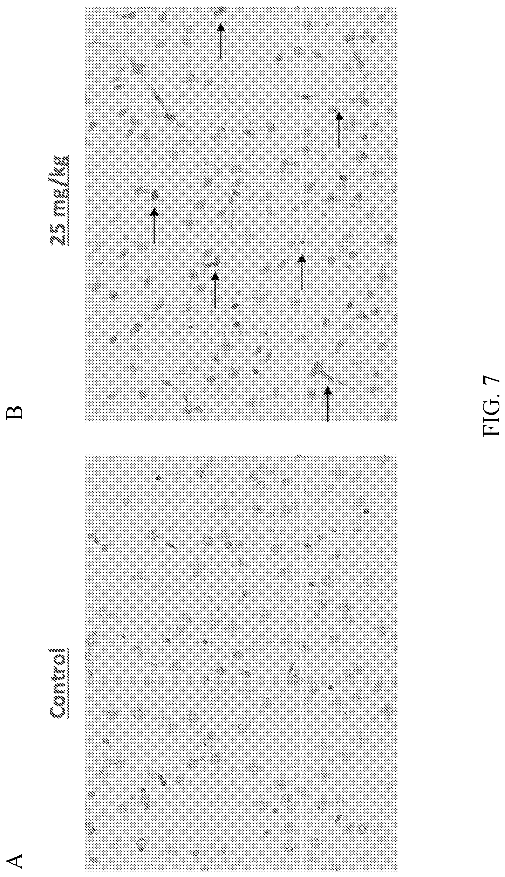

Brain sections from mice administered ST101 displayed evidence of cellular and microvascular staining for ST101 (**FIG. 7**, panel B), whereas there was no evidence of ST101 staining in brain sections from mice administered vehicle (**FIG. 7**, panel A). These data indicate that ST101 crosses the blood-brain barrier in naive C57BL/6 following systemic peptide administration.

### Example 4. ST101 Is Safe

### Safety Pharmacology

The potential pharmacological effects of ST101 on the central nervous, cardiovascular, and respiratory systems were investigated in a 28-day toxicity, toxicokinetic (TK) and safety pharmacology study under good laboratory practices (GLP) in cynomolgus monkeys. As described in greater detail below, no effects of ST101 on any of these systems were observed.

An electrocardiogram (ECG) examination was performed pre-dose and near the last dose administration. Monkeys were sedated by intramuscular injection of 100 mg/mL ketamine hydrochloride at a dose of approximately 10 mg/kg, in an effort to minimize handling-related stress. ECGs were evaluated qualitatively and quantitatively (e.g., QT & HR; QTc) by a board-certified veterinary cardiologist. ECGs were recorded using leads I, II, III, aVR, aVL, and aVF. Evaluation of electrocardiograms revealed no ST101-related changes.

For ancillary ECG and respiratory evaluation, ECG waveforms and waveforms from thoracic and abdominal expansions during ventilation were used to compare qualitative electrocardiographic and ventilation changes induced in animals that were administered either control (0 mg/kg) or ST101 at 30 mg/kg via IV bolus. Overall, ECG tracings from a total of 16 (8 males and 8 females; n=4/group/sex) monkeys were evaluated. For qualitative evaluation, the tracings from each animal were visually inspected for rhythm and/or morphology abnormalities in the ECG, and thoracic and abdominal respiratory leads. The qualitative analysis evaluated, among other factors, the interpretive quality of the tracings, the presence or absence of muscle tremor or 60 Hz artifacts, general alterations of voltages over time, configuration of ST-T, and rhythm. The data were compared between control animals and high dose animals, as well as between post-administration and baseline for control and high dose groups.

All monkeys had (usually) brief periods of recording where ECGs and pneumograms could be analyzed subjectively. There appeared to be no subjective changes in either electrocardiograms or pneumograms attributable to ST101.

Indirect funduscopic examination was performed by a board-certified veterinary ophthalmologist on sedated monkeys prior to assignment to study and towards the end of the treatment period. Evaluation of group mean ophthalmology revealed no ST101-related changes.

Respiration was recorded from monkeys by a staff veterinarian or trained designee during the acclimation period (baseline prior to treatment) and near the end of the treatment and recovery periods. The number of respirations was recorded for a 30 second interval and then multiplied by 2 to arrive at the respirations/minute. Evaluation of group mean respiration revealed no ST101-related changes.

Blood pressure (systolic, diastolic and mean arterial pressure) was collected on monkeys prior to assignment to study and near the end of the treatment and recovery periods. Blood pressure was collected concurrently with respiration examinations. Monkeys were sedated, in an effort to minimize handling-related stress. Evaluation of group mean blood pressure revealed no ST101-related changes.

### Assessment of Pseudoallergic Reactions

Clinical signs of pseudoallergic reaction can include decreased body temperature, reduced activity, and piloerection. No clinical signs of pseudoallergic reaction were observed in cynomolgus monkeys following intravenous (IV) bolus administration over 2-3 minutes of 10 mg/kg ST101, whereas administration of 30 mg/kg resulted in moderate hypoactivity that animals recovered from within 24 H. Extending the infusion rate from 2-3 minutes to 1-2 H resulted in a dose of 30 mg/kg ST101 that was well tolerated.

IV infusion of 50 mg/kg over 1 H or of 30 mg/kg over 30 minutes resulted in hypoactivity and flushing, which animals recovered from within hours. Subsequent studies demonstrated no signs of pseudoallergy in monkeys at dose levels of 7.5, 15 and 30 mg/kg administered by 1 H infusion 4 times over an 11-day period (n=2/sex/group).

In a 28-day GLP toxicity study in monkeys (*see* Example 5), 1 of 6 females and no males displayed signs of pseudoallergic reaction after the first exposure to ST101 in the 30 mg/kg group. This animal appeared to be recovering, as after several hours it began to eat and stir about, and intervention was not applied. Approximately 48 H after ST101 exposure, the animal's condition worsened, characterized by inactivity and a slow heartbeat, and the animal succumbed to death shortly thereafter. There were no other signs of pseudoallergic reaction in this study (n=4 core + 2 recovery monkey/sex/group, for a total of 36 ST101 treated animals). ST101 exposure levels tolerated in these studies are significantly greater than those to be tested in the clinic. Therefore, pseudoallergy is not expected to occur in human subjects.

### Example 5. ST101 Is Well-Tolerated

### Study 1

The tolerability of ST101 following repeated one-hour IV infusions at doses of 7.5 mg/kg, 15 mg/kg, and 30 mg/kg was evaluated in cynomolgus monkeys in a 12-day study. The study design, provided in **Table 1,** shows that each of the four groups (a group for each dose of ST101, and a vehicle control) consisted of four monkeys (two monkeys of each gender), and were administered ST101 or the vehicle control four times over 11 days.

**Table 1. Design for 12-Day Study in Cynomolgus Monkeys (Twice weekly dosing by 1-hour IV Infusion)**

| **Group** | **ST101 Dose [mg/kg/day]** | **Administration Schedule [study day]** | **Subjects [n]** | |
|---|---|---|---|---|
| | | | **Male** | **Female** |
| 1 | 0 (vehicle control) | 1, 4, 8, 11 | 2 | 2 |
| 2 | 7.5 | 1, 4, 8, 11 | 2 | 2 |
| 3 | 15 | 1, 4, 8, 11 | 2 | 2 |
| 4 | 30 | 1, 4, 8, 11 | 2 | 2 |

Blood specimens were collected on Day 1 and Day 11 at multiple time points prior to and after the dose administration. Tissues were collected from various organs including the brain, bladder, heart, intestines, kidneys, liver, and lungs, and were assessed and processed for evaluation.

The results indicated no significant changes in clinical observations, body weight, clinical chemistry, or hematology. Clear fluid was observed at one or more injection sites for both males receiving the 7.5 mg/kg-dose, one male receiving the 15 mg/kg-dose, one male receiving the 30 mg/kg-dose, and all the treated females. No other macroscopic findings relating to ST101 administration were observed.

Mild to marked thrombosis at the last injection site was noted for one male receiving the 7.5 mg/kg-dose, one male receiving the 15 mg/kg-dose, and both males receiving the 30 mg/kg-dose of ST101, and for all the treated females. No other microscopic ST101-related findings were observed.

Administration of the 7.5 mg/kg, 15 mg/kg, and 30 mg/kg doses in cynomolgus monkeys caused thrombosis, acute inflammation, accumulations of red blood cells, fibrosis, and edema at the injection sites. These findings were consistent with local irritation caused by administration of ST101. There were no other ST101-related macroscopic or microscopic findings.

Based on the results of this study, the dose of 30 mg/kg was considered to be the highest non-severely toxic dose (HNSTD) and the no-observed-adverse-effect-level (NOAEL) for ST101.

### Study 2

In this study, male and female cynomolgus monkeys were administered the test article ST101 at doses of 7.5, 15, and 30 mg/kg corresponding to Groups 2, 3, and 4, respectively, or the vehicle control (Group 1) as a one-hour intravenous (IV) infusion twice weekly, for a total of 8 doses. The last dose was administered on Day 24 for females and Day 25 for males. The study design, provided in **Table 2,** shows that each of the four groups (a group for each dose of ST101, and a vehicle control) consisted of eight monkeys (four of each gender) in the core study, plus four monkeys (two of each gender) in the recovery group. The recovery group underwent a two-week treatment-free period after the final exposure to ST101.

**Table 2. Design for 28-Day GLP Study in Cynomolgus Monkeys Followed by a 14-Day Recovery**

| **Group** | **ST101 Dose (mg/kg/day)** 1 H IV infusion | **Administration Schedule** | **Core Subjects** | |
|---|---|---|---|---|
| | | | **Males** | **Females** |
| 1 | 0 Control | Twice weekly x 4 weeks | 4+2 | 4+2 |
| 2 | 7.5 | Study days: 1, 4, 8, 11, 15, 18, 22, 25 | 4+2 | 4+2 |
| 3 | 15 | | 4+2 | 4+2 |
| 4 | 30 | | 4+2 | 4+2 |

Blood samples were collected on Day 1 and Day 24/25 for analysis of plasma concentrations of ST101 using a validated LC-MS/MS analytical method with a range of quantitation of 20.0 to 2000 ng/mL. The toxicokinetic (TK) parameters for ST101 were determined on Day 1 and Day 24/25 for Groups 2 (7.5 mg/kg), 3 (15 mg/kg), and 4 (30 mg/kg). Necropsy with gross observations and organ weight were performed on all animals, and histopathology was performed on all 30 mg/kg-dose and control core study animals.

Treatment-related observations included swelling/edema, scabbing, scaly skin, and sore/ulceration of the injection sites. The severity and incidence of these observations increased proportionate to dose level. Evaluation of group mean body weights and body weight gains revealed no ST101-related changes.

Clinical chemistry changes related to ST101 only occurred in the 30 mg/kg-dose group and were not observed in the 7.5 mg/kg-dose or the 15 mg/kg-dose groups. Similarly, hematological changes such as increased platelets and increased fibrinogen were limited to the 30 mg/kg-dose group. The increased platelet and fibrinogen levels as well as the increased creatinine phosphokinase correlate to the vascular irritation of the injection sites.

Evaluation of conventional ECG and respiratory data collected utilizing the DSI Jacketed (non-invasive) External Telemetry System with chest bands revealed no treatment-related changes.

ST101-related changes in organ weight parameters included increased liver and kidney (absolute and relative to brain and body weight) weights at the terminal necropsy in the 30 mg/kg-dose males and increased liver and kidney (absolute and relative to body weight) weights at the terminal necropsy in the 30 mg/kg-dose females. Absolute adrenal and kidney weights were increased for 15 mg/kg-dose females. Increased liver and kidney (absolute and relative to brain and body weight) weights and decreased thymus (absolute and relative to brain and body weight) weights were still evident in the 30 mg/kg-dose animals at the recovery necropsy.

Upon microscopic examination, minimal to moderate tubular necrosis of the kidney was noted for two males and three females given 30 mg/kg ST101, and a mild proteinaceous cast of the renal tubules was noted for one female given 30 mg/kg ST101. Minimal to marked tubular necrosis of the kidney was noted for two males and one female given 15 mg/kg. For recovery animals, minimal to mild tubular necrosis was noted in the kidneys of one 7.5 mg/kg-dose male, two 15 mg/kg-dose males, one 30 mg/kg-dose male and one 15 mg/kg-dose female. The tubular changes were partially reversible in the time period allotted for this study. The frequency and severity of the kidney lesions did not demonstrate a clear relationship to the administered dose, occurring sporadically at low severity in the core and recovery animals.

At terminal necropsy, gross lesions included clear fluid noted at one or more injection sites for two of the four males and two of the four females given 30 mg/kg. Crust/lesion/red pigmentation at one or more injection sites was noted for one of the four males and one of the four females given 7.5 mg/kg, three of the four males and one of the four females given 15 mg/kg and three of the four males and all females given 30 mg/kg. Mild to marked thrombosis at the last injection site was also noted for some animals in each dose group, occurring at a greater frequency in the 30 mg/kg-dose group. At recovery necropsy, crust/lesion/red pigmentation at one or more injection sites was noted for one of the two females given 15 mg/kg and both males given 30 mg/kg.

In light of these results, the HNSTD was considered to be the 15 mg/kg dose, and the NOAEL was considered to be the 7.5 mg/kg dose.

### Example 6: Administration of ST101 to Patients with Solid Tumors

An open-label phase 1-2 dose-finding study is conducted to determine the safety, tolerability, PK, pharmacodynamics (PD), and proof-of-concept efficacy of ST101 administered intravenously in patients with advanced unresectable and metastatic solid tumors. The study consists of two phases: (1) Dose Escalation Phase, and (2) Expansion Phase.

### Dose Escalation Phase

### Study Design

The Dose Escalation Phase involves the intravenous administration of ST101 to patients who are diagnosed with locally advanced or metastatic melanoma, carcinoma, or sarcoma of any tumor type who are refractory or intolerant to all available therapies that would impact survival. The dose cohorts are 0.5, 1, 2, 4, 6, 8, and 16 mg/kg, dosed once weekly (QW) in all cohorts; the highest dose level may be dosed every other week (Q2W). Administration is via IV infusion for a total infusion duration of at least 90 minutes. ST101 is provided in a pharmaceutical composition comprising trehalose and lactic acid, diluted in 0.9% saline.

Infusion-related reactions (IRR) may occur during and/or following the administration of ST101. Such reactions may include fevers, chills/rigors, tachycardia, tachypnea, hypotension, bronchospasm, and other recognized symptoms. IRR will not be regarded as a DLT unless relevant symptoms last more than two days. IRR will be used by the DRC to assess feasibility of the administration route and duration.

Supportive care measures may be performed to maintain the comfort of the patient through standard medical management. Such measures include stopping or slowing infusion, diphenhydramine, acetaminophen, and NSAIDs, crystalloid fluids, oxygen, bronchodilators, and other medications as clinically necessary, for example, as follows:
a. acetaminophen/paracetamol, 650-1000 mg, by IV or oral (PO) administration;
b. diphenhydramine, 25-50 mg, by IV or PO administration;
c. chlorphenamine, 5-10 mg, by IV or PO administration;
d. ranitidine, 50 mg, by IV or PO administration;
e. ibuprofen, 200-400 mg, by PO administration;
f. naproxen, 500 mg, by PO administration;
g. normal saline 0.9%, 1L or PRN, by IV (warmed) administration;
h. prochlorperazine, 5-10 mg, by IV or PO administration;
i. ondansetron, 4-16 mg, by IV or PO administration;
j. dexamethasone, 5-10 mg, by IV administration;
k. hydrocortisone, 100 mg once, by IV administration;
l. hydrocortisone, 50 mg q6h, by IV administration.

Patients experiencing relevant IRR at the first infusion receive prophylaxis at all subsequent infusions, with a minimum of diphenhydramine and acetaminophen, which should be administered before ST101 administration in anticipation of an IRR occurring during infusion.

### Results

The Dose Escalation Phase is ongoing. All patients in Cohorts 1-4 (dose levels 0.5, 1, 2, and 4 mg/kg, respectively; n=3 for each of Cohorts 1-3; n=6 for Cohort 4) have completed the 21-day DLT period, except that one patient in Cohort 4 did not receive all doses due to disease progression. In Cohort 5 (6 mg/kg), one patient has completed the DLT period and two patients are in the DLT period. Patients in Cohort 5 are administered 10 mg montelukast PO 2 days before and on the day of dosing, 20 mg famotidine PO or IV on the day of dosing, and 10 mg chlorphenamine PO or 50 mg diphenhydramine IV on the day of dosing. Patients with other conditions, such as allergies, can be administered a secondary agent, such as montelukast, daily.

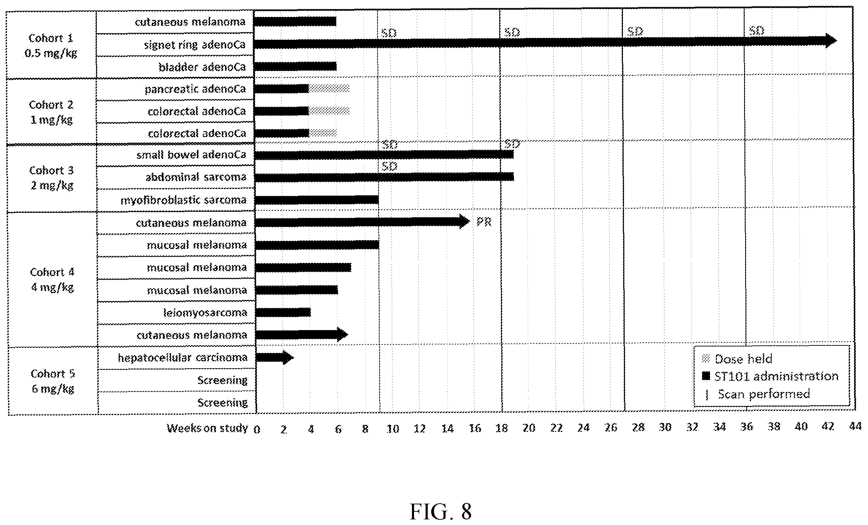

One patient in Cohort 1 and two patients in Cohort 3 have exhibited stable disease, as determined by a radiological assessment of the tumor (between 30% decrease and 20% increase in size), through at least 18 weeks. Notably, the patient in Cohort 1 has exhibited stable disease through 46 weeks. Status of disease in patients is shown in **FIG. 8****.**

A 62-year-old woman with metastatic cutaneous melanoma refractory to all available therapies reached Partial Response after 9 weeks of treatment with 4 mg/kg ST101. The patient previously experienced four local recurrences, which were resected and treated with isolated limb perfusion of melphalan. Metastasis to the lung was treated with ipilimumab-nivolumab combination therapy then nivolumab monotherapy, which was stopped due to hypophysitis. Subsequent frontal lobe brain metastasis was treated with complete resection, radiotherapy, and nivolumab until Progressive Disease PD. Subsequent miliary lung metastasis was treated with temozolomide for approximately 16 months until PD, following resection of a large abdominal mass. The patient was diagnosed with piriformis metastasis shortly before beginning the present study. Assessment of the patient's response to ST101 therapy, measured by CT with contrast is shown in **Table 3.**

**Table 3. Tumor Response in Melanoma Patient**

| **Target Lesions*** | **Baseline** | **Week 9** | **Week 15** |
|---|---|---|---|
| Lung LUL | 12 | 15 | 12 |
| Lung LLL | 11 | 6 | 6 |
| Pelvis soft tissue L piriformis | 36 | 19 | 18 |
| Abdomen L rectus | 15 | 12 | 10 |
| Sum of target lesions | 74 | 52 | 46 |
| % change from baseline | - | **30%** | **38%** |

| **Non-target lesions** | | | |
|---|---|---|---|
| Multiple scattered nodules both lungs | Present | **Decreased** | **Decreased+** |
| New lesions | - | none | none |
| **Overall response** | - | **PR** | **PR** |

| | | | |
|---|---|---|---|
| *Longest diameter (mm); +compared to baseline, not week 9; PR = Partial Response | | | |

All except for one of the patient's lesions shrank, with the largest lesion showing substantial shrinkage. In addition, non-target multiple lung lesions decreased in size and no new lesions were detected.

Most of the AEs experienced by the patients were IRRs that occurred in the first six hours and that were controlled with antihistamines/anti-inflammatory agents. To manage IRR, the following measures were used in the patients: antihistamines (58% of the 12 patients), NSAIDs (17%, none by patients in Cohorts 1, 3, or 4), acetaminophen/paracetamol (50%), corticosteroids (42%, none by patients in Cohorts 1 or 2), famotidine (8%, none by patients in cohorts 1, 2 or 4), interrupted infusion (42%, none by patients in Cohorts 1 or 2), and slowed infusion (42%, none by patients in Cohorts 1 or 2).

Blood samples were drawn pre-dose and at various timepoints after the total infusion period during the first cycle and less frequently thereafter. For patients who were in the QW dosing cohorts and were therefore following a 21-day treatment cycle, blood samples were drawn at the following time points shown in **Table 4.**

**Table 4. Timepoints for Blood Draws in QW Cohorts**

| **Cycle** | **Day** | **Time Points** |
|---|---|---|
| 1 | 1 | ▪ End of total infusion period |
| | | ▪ 4 hours post-Day 1 infusion |
| | | ▪ 8 hours post-Day 1 infusion |
| | 2 | ▪ 24 hours post-Day 1 infusion |
| | 3 | ▪ 48 hours post-Day 1 infusion |
| | 4 | ▪ 72 hours post-Day 1 infusion |
| | 8 | ▪ 4 hours post-Day 8 infusion |
| | 15 | ▪ 4 hours post-Day 15 infusion |
| 2 | 1 | ▪ Prior to infusion |
| | | ▪ End of total infusion period |
| | | ▪ 4 hours post-Day 1 infusion |
| | | ▪ 8 hours post-Day 1 infusion |
| | 2 | ▪ 24 hours post-Day 1 infusion, if patient is hospitalized overnight |
| | 8 | ▪ Prior to infusion |
| | | ▪ 4 hours post-Day 8 infusion |
| 3+ | 1 | ▪ Prior to infusion |
| | | ▪ 4 hours post-Day 1 infusion |

For patients who are in the Q2W dosing cohorts and are therefore following a 28-day treatment cycle, blood samples are drawn at the following time points shown in **Table 5.**

**Table 5. Timepoints for Blood Draws in Q2W Cohorts**

| **Cycle** | **Day** | **Time Points** |
|---|---|---|
| 1 | 1 | ▪ End of total infusion period |
| | | ▪ 4 hours post-Day 1 infusion |
| | | ▪ 8 hours post-Day 1 infusion |
| | 2 | ▪ 24 hours post-Day 1 infusion |
| | 15 | ▪ 4 hours post-Day 15 infusion |
| 2 | 1 | ▪ End of total infusion period |
| | | ▪ 4 hours post-Day 1 infusion |
| | | ▪ 8 hours post-Day 1 infusion |
| | 2 | ▪ 24 hours post-Day 1 infusion, if patient is hospitalized overnight |
| 3+ | 1 | ▪ 4 hours post-Day 1 infusion |

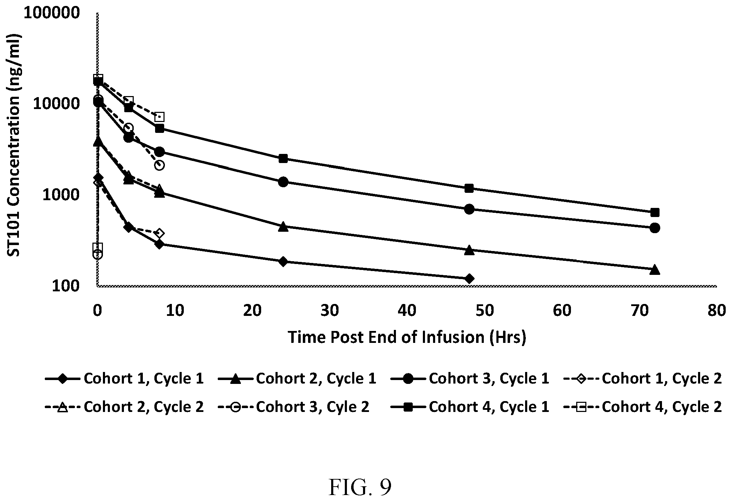

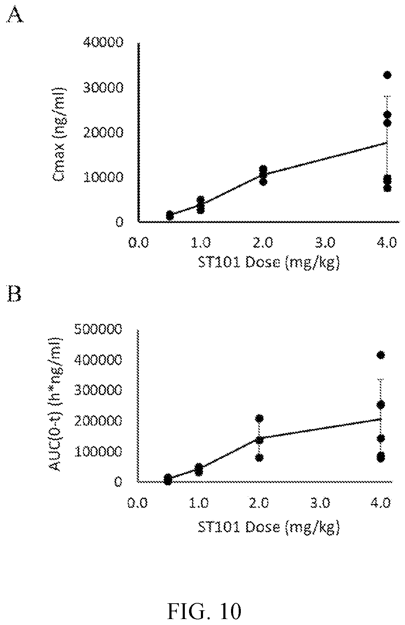

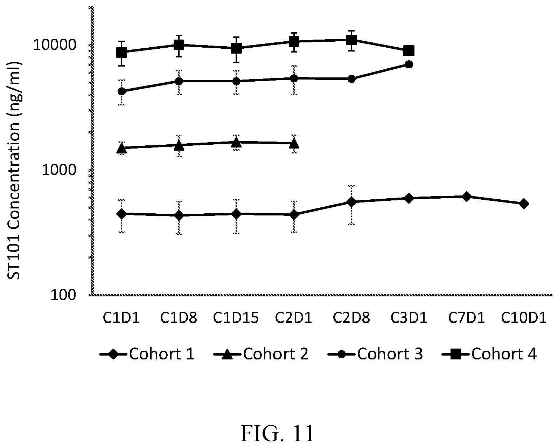

ST101 pharmacokinetics (PK) in plasma were assessed via liquid chromatography coupled to tandem mass spectrometry (LC-MS). The PK results for Cohorts 1-4 showed that Cₘₐₓ and AUCₜ were dose-proportional (**FIG. 9****;** **FIG. 10****,** panels A and B), and each cohort showed minimal accumulation of ST101 at four hours post-infusion (**FIG. 11**). In addition, Tₘₐₓ for each cohort was at or near the end of infusion, although mean elimination half-life differed among the cohorts. PK results are summarized in **Table 6.**

**Table 6. Summary of Pharmacokinetic Parameters of Cycle 1**

| **Parameter** | **Cohort 1 (n=3)** | | **Cohort 2 (n=3)** | | **Cohort 3 (n=3)** | | **Cohort 4 (n=3)** | |
|---|---|---|---|---|---|---|---|---|
| | **Mean** | **SD** | **Mean** | **SD** | **Mean** | **SD** | **Mean** | **SD** |
| Cₘₐₓ (ng/mL) | 1560 | 201 | 3890 | 1100 | 10500 | 1450 | 17700 | 10300 |
| Tₘₐₓ (h) | 1.66 | 0.207 | 1.63 | 0.136 | 2.78 | 1.33 | 1.22 | 1.81 |
| AUC₀₋ₜ (h*ng/mL) | 10400 | 5850 | 42000 | 7610 | 143000 | 64400 | 206000 | 129000 |
| Tₗₐₛₜ (h) | 28.2 | 20 | 72.9 | 0.5 | 135 | 55.5 | 117 | 59 |
| AUC₀₋₈ (h*ng/mL) | 5710 | 1610 | 16300 | 3790 | 46300 | 11500 | 68500 | 33600 |
| AUC_{0-inf} (h*ng/mL) | 17100* | 3940* | 48400 | 8560 | 154000 | 64400 | 227000 | 139000 |
| t_{½} (h) | 18* | 9.56* | 28.7 | 1.79 | 41.2 | 11.7 | 37.8 | 15.8 |
| Clearance (L/h) | 2.5* | 1.23* | 1.68 | 0.512 | 1.21 | 0.38 | 1.73 | 0.70 |
| V_{z}(L) | 56.5* | 2.6* | 70.5 | 26.2 | 69.9 | 20.6 | 95.6 | 57.2 |

| | | | | | | | | |
|---|---|---|---|---|---|---|---|---|
| *calculated based on n=2 | | | | | | | | |

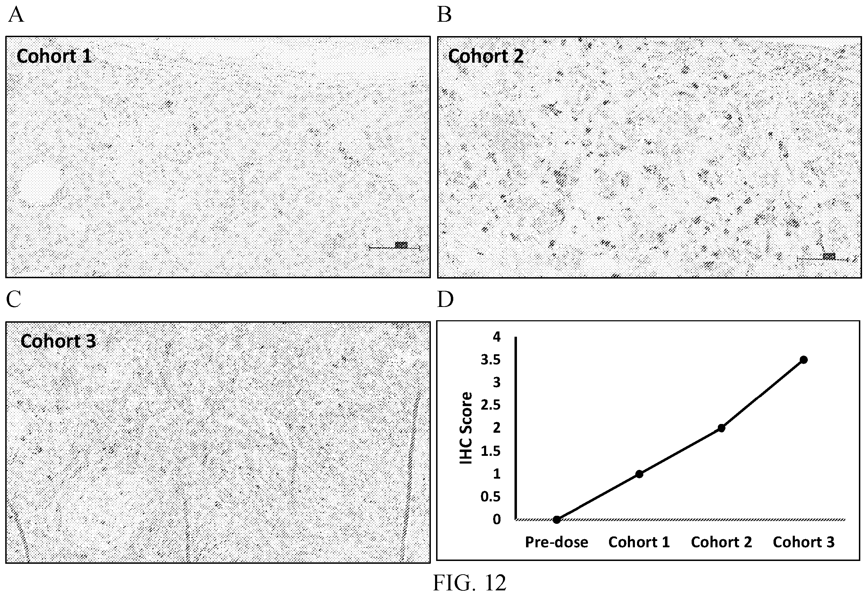

Tumor uptake of ST101 was determined by immunohistochemistry analysis. Biopsy samples collected from patients during screening, *i.e.,* prior to treatment with ST101, and during cycle 2 of treatment were processed and immunoassayed for ST101 using rabbit polyclonal anti-ST101 antibody. Sections were analyzed by a Board-certified pathologist and scored based on prevalence of cells displaying ST101 uptake. Data indicates that ST101 uptake into tumors increases with increasing ST101 dose (**FIG. 12**). All biopsies collected from patients during screening, prior to ST101 exposure, were negative for ST101 immunostaining.

The impact of ST101 on tumor cell proliferation was determined by immunohistochemistry analysis. Biopsy samples collected from patients during screening and cycle 2 of treatment were processed and immunoassayed for presence of Ki67. Sections were analyzed by a Board-certified pathologist and scored based on intensity of Ki67 signal. Data indicates that Ki67 signal decreased following ST101 exposure, as evidenced by decreased Ki67 staining (**FIG. 13**).

Pharmacodynamics assessments include circulating cell-free deoxynucleic acid (DNA) analysis for mechanistically associated gene abnormalities and changes over time, and tumor analysis at baseline and on treatment using quantitative reverse transcriptase (qRT)-polymerase chain reaction (PCR), Nanostring, and immunohistochemistry (IHC).

### Expansion Phase

The study proceeds to the Expansion Phase once the highest escalation cohort is reached or a notable efficacy is observed at a given dose level, and a recommended phase 2 dose (RP2D) is selected. Selection of the RP2D is based on the safety, PK, PD, and efficacy data. The RP2D could be the maximum tolerated dose (MTD), the "active dose" (dose of ST101 which is considered to have anti-tumor activity, selected based on preclinical, safety, PK, PD, and efficacy data), or an alternative dose between the MTD and active dose. The Expansion Phase consists of four specific tumor-type cohorts, described below.

At least 10 patients in the Expansion Phase cohorts have a mandatory core or excisional biopsy at screening, within 28 days before enrollment. Post-treatment biopsy of the same lesion is performed, if feasible, and if tumor persists in patients who had a screening biopsy. An additional optional biopsy of any other lesion can be performed.

### Administration of ST101 to Breast Cancer Patients

Patients diagnosed with hormone receptor-positive (HR^{pos}), locally advanced or metastatic breast cancer (LA/MBC) that has progressed after prior one-to-two hormone-based therapies are administered a dose of 0.5, 1, 2, 4, 6, 8, 12, or 16 mg/kg ST101 once weekly, or 16 mg/kg ST101 every other week. Previous treatment with cyclin-dependent kinase 4/6 (CDK4/6) inhibitor, mammalian target of rapamycin (mTOR) inhibitor, or chemotherapy is allowed as monotherapy or in combination.

RECIST 1.1 (Eisenhauer 2009) is used to assess tumor response, disease control rate (DCR), duration of response (DOR), and/or progression-free survival (PFS). Either computed CT or MRI is utilized to assess tumor response, with CT being the preferred imaging technique. A radiological assessment of a CR or PR requires confirmatory imaging at least 4 weeks after the initial assessment of response was observed. A radiological assessment of SD requires confirmatory imaging at least 6 weeks after the initial assessment of response was observed. PFS is determined from the time of first treatment administration to the first documented disease progression or death. DOR is determined from the time of first observed response to the first documented disease progression or death.

### Administration of ST101 to Melanoma Patients

Patients diagnosed with advanced/metastatic melanoma that has progressed after or on treatment with an immune checkpoint inhibitor (CPI) and have received one-to-two prior lines of therapy are administered a dose of 0.5, 1, 2, 4, 6, 8, 12, or 16 mg/kg ST101 once weekly, or 16 mg/kg ST101 every other week. Patients that have BRAF mutated disease should also have received one line of appropriate targeted therapy.

RECIST 1.1 (Eisenhauer 2009) is used to assess tumor response, disease control rate (DCR), duration of response (DOR), and/or progression-free survival (PFS). Either computed CT or MRI is utilized to assess tumor response, with CT being the preferred imaging technique. A radiological assessment of a CR or PR requires confirmatory imaging at least 4 weeks after the initial assessment of response was observed. A radiological assessment of SD requires confirmatory imaging at least 6 weeks after the initial assessment of response was observed. PFS is determined from the time of first treatment administration to the first documented disease progression or death. DOR is determined from the time of first observed response to the first documented disease progression or death.

### Administration of ST101 to Glioblastoma Patients

Patients diagnosed with primary (de novo) GBM that has recurred or progressed (per modified RANO criteria) after one standard treatment regimen are administered a dose of 0.5, 1, 2, 4, 6, 8, 12, or 16 mg/kg ST101 once weekly, or 16 mg/kg ST101 every other week. Standard therapy is defined as maximal surgical resection, radiotherapy, and concomitant temozolomide with radiotherapy or adjuvant chemotherapy with temozolomide. Patients that undergo tumor treating fields as an adjuvant to first line therapy are eligible.

Modified RANO (Ellingson 2017) is used to assess tumor response, disease control rate (DCR), duration of response (DOR), and/or progression-free survival (PFS). Gadolinium-enhanced MRI is utilized to assess tumor response. A radiological assessment of a CR or PR requires confirmatory imaging at least 4 weeks after the initial assessment of response was observed. A radiological assessment of SD requires confirmatory imaging at least 6 weeks after the initial assessment of response was observed. PFS is determined from the time of first treatment administration to the first documented disease progression or death. DOR is determined from the time of first observed response to the first documented disease progression or death.

### Administration of ST101 to Prostate Cancer Patients

Patients diagnosed with castration-resistant prostate cancer (CRPC) that has progressed after previous treatment with taxanes, abiraterone and enzalutamide/apalutamide (unless contraindicated or the patients were intolerant to these agents) are administered a dose of 0.5, 1, 2, 4, 6, 8, 12, or 16 mg/kg ST101 once weekly, or 16 mg/kg ST101 every other week.

Responses are graded using PCWG3 guidelines (Scher 2016). Either CT or MRI is utilized to assess tumor response. Imaging assessment is performed until progressive disease (PD). A radiological assessment of a CR or PR requires confirmatory imaging at least 4 weeks after the initial assessment of response was observed. A radiologic assessment of stable disease (SD) requires confirmatory imaging at least 6 weeks after the initial assessment of response was observed. The assessments for DCR, DOR and PFS are based on PCWG3 measurements. The time of first study treatment administration to the first documented disease progression or death will determine PFS. The time of first observed response to the first documented disease progression or death will determine DOR.

### REFERENCES

Aguilar-Morante D, et al. Decreased CCAAT/enhancer binding protein beta expression inhibits the growth of glioblastoma cells. Neuroscience 176:110-119 (2011).
Angelastro JM, et al. Selective destruction of glioblastoma cells by interference with the activity or expression of ATF5. Oncogene 2006; 25: 907-916.
Asada R, et al. The signalling from endoplasmic reticulum-resident bZIP transcription factors involved in diverse cellular physiology. J Biochem 2011; 149: 507-518.
Bernal F, et al. Reactivation of the p53 Tumor Suppressor Pathway by a Stapled p53 Peptide. J. Am. Chem. Soc. 2007; 129:2456-2457.
Bezy O, et al. Delta-interacting protein A, a new inhibitory partner of CCAAT/enhancer-binding protein beta, implicated in adipocyte differentiation. J Biol Chem 2005; 280: 11432-11438.
Bird GH, et al. Biophysical Determinants for Cellular Uptake of Hydrocarbon-Stapled Peptide Helices. Nat. Chem. Biol. 2017; 12:845-852.
Bruzzoni-Giovanelli H, et al. Interfering peptides targeting protein-protein interactions: the next generation of drugs? Drug Discov Today 2018; 23: 272-285.
Bushweller JH. Targeting transcription factors in cancer - from undruggable to reality. Nat Rev Cancer 2019; 19: 611-624.
Cates CC, et al. Regression/eradication of gliomas in mice by a systemically-deliverable ATF5 dominant-negative peptide. Oncotarget 2016; 7: 12718-12730.
Demma MJ, et al. Omomyc Reveals New Mechanisms To Inhibit the MYC Oncogene. Mol Cell Biol 2019; 39.
Dougherty PG, et al. Understanding Cell Penetration of Cyclic Peptides. Chem. Rev. 2019; 119(17):10241-10287.
Eisenhauer EA, et al. New response evaluation criteria in solid tumours: Revised RECIST guideline (version 1.1). Eur J Cancer 2009; 45(2):228-247.
Ellingson B, et al. Modified Criteria for Radiographic Response Assessment in Glioblastoma Clinical Trials. Neurotherapeutics. 2017; 14:307-320.
Homma J, et al. Increased expression of CCAAT/enhancer binding protein beta correlates with prognosis in glioma patients. Oncol Rep 2006; 15: 595-601.
Huggins CJ, et al. C/EBPgamma suppresses senescence and inflammatory gene expression by heterodimerizing with C/EBPbeta. Mol Cell Biol 2013; 33: 3242-3258.
Karpel-Massler G, et al. A Synthetic Cell-Penetrating Dominant-Negative ATF5 Peptide Exerts Anticancer Activity against a Broad Spectrum of Treatment-Resistant Cancers. Clin Cancer Res 2016; 22: 4698-4711.
Kim MH, et al. Translationally regulated C/EBP beta isoform expression upregulates metastatic genes in hormone-independent prostate cancer cells. Prostate 68:1362-1371 (2008).
Lamb J, et al. A mechanism of cyclin D1 action encoded in the patterns of gene expression in human cancer. Cell 2003; 114: 323-334.
Lambert M, et al. Targeting Transcription Factors for Cancer Treatment. Molecules 2018; 23.
Lathbridge A, et al. Computational Competitive and Negative Design To Derive a Specific cJun Antagonist. Biochemistry 2018; 57: 6108-6118.
Lathbridge A, et al. Coupling Computational and Intracellular Screening and Selection Toward Co-compatible cJun and cFos Antagonists. Biochemistry 2019.
Lekstrom-Himes J, et al. Biological role of the CCAAT/enhancer-binding protein family of transcription factors. J Biol Chem 1998; 273: 28545-28548.
Oya M, et al. Increased activation of CCAAT/enhancer binding protein-beta correlates with the invasiveness of renal cell carcinoma. Clin Cancer Res 2003; 9: 1021-1027.
Pal R, et al. C/EBPbeta regulates transcription factors critical for proliferation and survival of multiple myeloma cells. Blood 114:3890-3898 (2009).
Potapov V, et al. Data-driven prediction and design of bZIP coiled-coil interactions. PLoS Comput Biol 2015; 11: e1004046.
Scher HI, et al. End Points and Outcomes in Castration-Resistant Prostate Cancer: From Clinical Trials to Clinical Practice. J. Clin. Oncology. 2011:29(27):3695-3704.
Scher HI, et al. Trial Design and Objectives for Castration-Resistant Prostate Cancer: Updated Recommendations from the Prostate Cancer Clinical Trials Working Group 3. J Clin Oncology. 2016:34(12):1402-1418.
Sheng Z, et al. An activating transcription factor 5-mediated survival pathway as a target for cancer therapy? Oncotarget 2010; 1: 457-460.
Takada K, et al. Targeted disruption of the BCL9/beta-catenin complex inhibits oncogenic Wnt signaling. Sci Transl Med 2012; 4: 148ra117.
Walensky LD, et al. Hydrocarbon-stapled peptides: principles, practice, and progress. J Med Chem 2014; 57: 6275-6288.
Yan C, et al. Drugging the undruggable: transcription therapy for cancer. Biochim Biophys Acta 2013; 1835: 76-85.
Zahnow CA. CCAAT/enhancer-binding protein beta: its role in breast cancer and associations with receptor tyrosine kinases. Expert Rev Mol Med 2009; 11: e 12.
Zhang ZY, et al. Stabilization of ATF5 by TAK1-Nemo-like kinase critically regulates the interleukin-1beta-stimulated C/EBP signaling pathway. Mol Cell Biol 2015; 35: 778-788.
Zhao Y, et al. p300-dependent acetylation of activating transcription factor 5 enhances C/EBPbeta transactivation of C/EBPalpha during 3T3-L1 differentiation. Mol Cell Biol 2014; 34: 315-324.

## Claims

1. A pharmaceutical composition comprising a peptide antagonist of CCAAT-enhancer-binding protein β (C/EBPβ) for use in treating a solid tumor in a human patient, wherein the peptide antagonist consists of the D-amino acid sequence VAEAREELERLEARLGQARGELKKWKMRRNQFWLKLQR (SEQ ID NO:2), wherein the pharmaceutical composition is to be administered via intravenous infusion, and wherein the peptide antagonist is to be administered at a dose of 0.5-16 mg/kg.

2. The pharmaceutical composition for the use of claim 1, wherein the solid tumor is a melanoma, a carcinoma, or a sarcoma.

3. The pharmaceutical composition for the use of claim 1 or 2, wherein the patient has been diagnosed with locally advanced or metastatic breast cancer (LA/MBC), melanoma, glioblastoma (GBM), or castration-resistant prostate cancer (CRPC).

4. The pharmaceutical composition for use of any one of claims 1 to 3, wherein one or more secondary agents selected from the group consisting of antihistamines, leukotriene inhibitors, nonsteroidal anti-inflammatory drugs, acetaminophen, corticosteroids, antinausea medications, intravenous saline, and electrolytes are to be administered concurrently with, before, or after administration of the pharmaceutical composition.

5. The pharmaceutical composition for the use of claim 4, wherein an antihistamine and/or a leukotriene inhibitor is to be administered to said patient within about 48 hours prior to administration of the pharmaceutical composition.

6. The pharmaceutical composition for the use of claim 3 for the treatment of melanoma, wherein the patient has received at least one line of therapy prior to administration of the pharmaceutical composition.

7. The pharmaceutical composition for the use of claim 6, wherein
(i) the patient has melanoma that has progressed after or on treatment with an immune checkpoint inhibitor, and wherein the patient has advanced/metastatic melanoma; or
(ii) the patient has melanoma comprising a BRAF mutation, and wherein the patient has received at least one line of targeted therapy.

8. The pharmaceutical composition for the use of claim 3 for the treatment of GBM, wherein the GBM has recurred or progressed after previous treatment by surgery; radiation; chemotherapy, preferably with temozolomide, carmustine, or lomustine; targeted therapy; electric field therapy; or combinations thereof.

9. The pharmaceutical composition for the use of claim 3 for the treatment of CRPC, wherein the CRPC has progressed after previous treatment with a taxane, abiraterone, daralutamide, and/or enzalutamide/apalutamide.

10. The pharmaceutical composition for the use of any one of claims 1 to 9, wherein the pharmaceutical composition is to be administered once weekly or once every two weeks.

11. The pharmaceutical composition for the use of any one of claims 1 to 10, wherein the pharmaceutical composition is to be administered for at least three weeks or at least four weeks.

12. The pharmaceutical composition for the use of any one of claims 1 to 11, wherein the pharmaceutical composition is to be administered by intravenous infusion for a total infusion duration of about 30 minutes to about 360 minutes or of about 60 minutes to about 360 minutes.

## Patentansprüche

1. Pharmazeutische Zusammensetzung, die einen Peptidantagonist von CCAAT-Enhancer-Bindungsprotein β (CCAAT-enhancer binding protein β; C/EBPβ) umfasst, zur Verwendung bei der Behandlung eines soliden Tumors in einem humanen Patienten, wobei der Peptidantagonist aus der D-Aminosäuresequenz VAEAREELERLEARLGQARGELKKWKMRRNQFWLKLQR (SEQ ID NO:2) besteht, wobei die pharmazeutische Zusammensetzung über intravenöse Infusion zu verabreichen ist, und wobei der Peptidantagonist mit einer Dosis von 0,5 - 16 mg/kg zu verabreichen ist.

2. Pharmazeutische Zusammensetzung zur Verwendung nach Anspruch 1, wobei der solide Tumor ein Melanom, ein Karzinom oder ein Sarkom ist.

3. Pharmazeutische Zusammensetzung zur Verwendung nach Anspruch 1 oder 2, wobei bei dem Patienten lokal fortgeschrittener oder metastasierter Brustkrebs (locally advanced or metastatic breast cancer; LA/MBC), Melanom, Glioblastom (GBM) oder kastrationsresistenter Prostatakrebs (castration-resistant prostate cancer; CRPC) diagnostiziert wurde.

4. Pharmazeutische Zusammensetzung zur Verwendung nach einem der Ansprüche 1 bis 3, wobei ein oder mehrere sekundäre Agenzien, die ausgewählt sind aus der Gruppe bestehend aus Antihistaminika, Leukotrien-Inhibitoren, nichtsteroidalen Entzündungshemmern, Acetaminophen, Kortikosteroiden, Antiemetika, intravenöser Kochsalzlösung und Elektrolyten, gleichzeitig mit, vor oder nach der Verabreichung der pharmazeutischen Zusammensetzung zu verabreichen sind.

5. Pharmazeutische Zusammensetzung zur Verwendung nach Anspruch 4, wobei ein Antihistamin und/oder ein Leukotrien-Inhibitor an den Patienten innerhalb etwa 48 Stunden vor der Verabreichung der pharmazeutischen Zusammensetzung zu verabreichen ist.

6. Pharmazeutische Zusammensetzung zur Verwendung nach Anspruch 3 zur Behandlung von Melanom, wobei der Patient mindestens eine Therapielinie vor der Verabreichung der pharmazeutischen Zusammensetzung erhalten hat.

7. Pharmazeutische Zusammensetzung zur Verwendung nach Anspruch 6, wobei
(i) der Patient ein Melanom hat, das nach oder während der Behandlung mit einem Immuncheckpoint-Inhibitor fortgeschritten ist, und wobei der Patient ein fortgeschrittenes/metastasiertes Melanom hat; oder
(ii) der Patient ein Melanom hat, das eine BRAF-Mutation umfasst, und wobei der Patient mindestens eine Linie gezielter Therapie erhalten hat.

8. Pharmazeutische Zusammensetzung zur Verwendung nach Anspruch 3 zur Behandlung von GBM, wobei das GBM nach vorheriger Behandlung durch Operation; Bestrahlung; Chemotherapie, bevorzugt mit Temozolomid, Carmustin oder Lomustin; gezielte Therapie; elektrische Feldtherapie; oder Kombinationen davon rezidiviert oder fortgeschritten ist.

9. Pharmazeutische Zusammensetzung zur Verwendung nach Anspruch 3 zur Behandlung von CRPC, wobei das CRPC nach vorheriger Behandlung mit einem Taxan, Abirateron, Daralutamid und/oder Enzalutamid/Apalutamid fortgeschritten ist.

10. Pharmazeutische Zusammensetzung zur Verwendung nach einem der Ansprüche 1 bis 9, wobei die pharmazeutische Zusammensetzung einmal wöchentlich oder einmal alle zwei Wochen zu verabreichen ist.

11. Pharmazeutische Zusammensetzung zur Verwendung nach einem der Ansprüche 1 bis 10, wobei die pharmazeutische Zusammensetzung mindestens drei Wochen lang oder mindestens vier Wochen lang zu verabreichen ist.

12. Pharmazeutische Zusammensetzung zur Verwendung nach einem der Ansprüche 1 bis 11, wobei die pharmazeutische Zusammensetzung durch intravenöse Infusion für eine Gesamtinfusionsdauer von etwa 30 Minuten bis etwa 360 Minuten oder von etwa 60 Minuten bis etwa 360 Minuten zu verabreichen ist.

## Revendications

1. Composition pharmaceutique comprenant un peptide antagoniste d'une protéine de liaison β à un enhancer de type CCAAT (C/EBPβ) pour une utilisation dans le traitement d'une tumeur solide chez un patient humain, dans laquelle le peptide antagoniste consiste en la séquence d'acides D-aminés VAEAREELERLEARLGQARGELKKWKMRRNQFWLKLQR (SEQ ID NO : 2), laquelle composition pharmaceutique est destinée à être administrée par perfusion intraveineuse, et dans laquelle le peptide antagoniste est destiné à être administré à une dose de 0,5 à 16 mg/kg.

2. Composition pharmaceutique pour une utilisation selon la revendication 1, dans laquelle la tumeur solide est un mélanome, un carcinome ou un sarcome.

3. Composition pharmaceutique pour une utilisation selon la revendication 1 ou 2, dans laquelle le patient a été diagnostiqué avec un cancer du sein métastasique ou localement avancé (LA/MBC), un mélanome, un glioblastome (GBM) ou un cancer de la prostate résistant à la castration (CRPC).

4. Composition pharmaceutique pour une utilisation selon l'une quelconque des revendications 1 à 3, dans laquelle un ou plusieurs agents secondaires choisis dans le groupe constitué par les antihistaminiques, les inhibiteurs de leucotriène, les anti-inflammatoires non stéroïdiens, l'acétaminophène, les corticostéroïdes, les médicaments antinauséeux, la solution saline à usage intraveineux, et les électrolytes, sont destinés à être administrés en même temps que, avant, ou après l'administration de la composition pharmaceutique.

5. Composition pharmaceutique pour une utilisation selon la revendication 4, dans laquelle un antihistaminique et/ou un inhibiteur de leucotriène sont destinés à être administrés audit patient dans les 48 heures environ avant l'administration de la composition pharmaceutique.

6. Composition pharmaceutique pour une utilisation selon la revendication 3 pour le traitement d'un mélanome, dans laquelle le patient a reçu au moins une ligne de thérapie avant l'administration de la composition pharmaceutique.

7. Composition pharmaceutique pour une utilisation selon la revendication 6, dans laquelle
(i) le patient a un mélanome qui a progressé après ou lors d'un traitement avec un inhibiteur du point de contrôle immunitaire, et le patient a un mélanome avancé/ métastasique ; ou
(ii) le patient a un mélanome comprenant une mutation BRAF ; et le patient a reçu au moins une ligne de thérapie ciblée.

8. Composition pharmaceutique pour une utilisation selon la revendication 3 pour le traitement d'un GBM, dans laquelle le GBM a récidivé ou progressé après un traitement antérieur par chirurgie ; irradiation ; chimiothérapie, de préférence au témozolomide, à la carmustine ou à la lomustine ; thérapie ciblée ; thérapie par champ électrique ; ou combinaisons de celles-ci.

9. Composition pharmaceutique pour une utilisation selon la revendication 3 pour le traitement d'un CRPC, dans laquelle le CRPC a progressé après un traitement antérieur avec un taxane, l'abiraténone, le daralutamide, et/ou l'enzalutamide/ apalutamide.

10. Composition pharmaceutique pour une utilisation selon l'une quelconque des revendications 1 à 9, laquelle composition pharmaceutique est destinée à être administrée une fois par semaine ou une fois toutes les deux semaines.

11. Composition pharmaceutique pour une utilisation selon l'une quelconque des revendications 1 à 10, laquelle composition pharmaceutique est destinée à être administrée pendant au moins trois semaines ou au moins quatre semaines.

12. Composition pharmaceutique pour une utilisation selon l'une quelconque des revendications 1 à 11, laquelle composition pharmaceutique est destinée à être administrée par perfusion intraveineuse pour une durée de perfusion totale d'environ 30 minutes à environ 360 minutes ou d'environ 60 minutes à environ 360 minutes.
